# EUROPEAN PATENT SPECIFICATION

(11) **EP 2 477 649 B1**
(45) Date of publication and mention of the grant of the patent: **23.11.2016**
(21) Application number: 10817520.9
(22) Date of filing: 16.09.2010
(51) Int. Cl.: A61K 39/106, A61K 39/108, A61P 31/04

(54) **VACCINE AGAINST CHOLERA AND ENTEROTOXIGENIC E. COLI (ETEC) DIARRHEA**
IMPFSTOFF GEGEN CHOLERA UND ENTEROTOXIGENE E. COLI (ETEC)-DIARRHÖE
VACCIN CONTRE LA DIARRHÉE DUE AU CHOLÉRA ET A E.COLI ENTÉROTOXIGÈNE (ETEC)

(30) Priority: 16.09.2009 US 272351 P
(43) Date of publication of application: 25.07.2012
(73) Proprietor: MSD Wellcome Trust Hilleman Laboratories Pvt Ltd., New Delhi 110062 (IN)
(72) Inventor: HOLMGREN, Jan, S-426 74 Västra Frölunda (SE); LEBENS, Michael, S-523 99 Hökerum (SE)
(74) Representative: Novitas Patent AB
(86) International application number: PCT/SE2010/050996
(87) International publication number: WO 2011/034495

(56) References cited:
- WO-A1-00/67784
- WO-A1-94/01533
- WO-A2-2009/049013
- US-A- 5 330 753
- US-A- 5 882 653
- US-A- 5 942 242
- US-A1- 2006 099 229
- US-B1- 6 203 799
- US-B2- 7 270 961
- SU L CHIANG ANDJOHN J MEKALANOS: "Construction of a Vibrio cholerae vaccine candidate using transposon delivery and FLP recombinase- mediated excision", INFECTION AND IMMUNITY, AMERICAN SOCIETY FOR MACROBIOLOGY, USA, vol. 68, no. 11, 1 January 2000 (2000-01-01), pages 6391-6397, XP008153886, ISSN: 0019-9567, DOI: 10.1128/IAI.68.11
- S. G RIJPKEMA ET AL: "Assessing clonality of Vibrio cholerae Inaba isolates by characterization of nonsense mutations in wbeT", JOURNAL OF MEDICAL MICROBIOLOGY, vol. 53, no. 11, 1 November 2004 (2004-11-01), pages 1105-1107, XP055048436, ISSN: 0022-2615, DOI: 10.1099/jmm.0.45744-0
- KANUNGO S ET AL: "Immune responses following one and two doses of the reformulated, bivalent, killed, whole-cell, oral cholera vaccine among adults and children in Kolkata, India: A randomized, placebo-controlled trial", VACCINE, ELSEVIER LTD, GB, vol. 27, no. 49, November 2009 (2009-11), pages 6887-6893, XP026722082, ISSN: 0264-410X, DOI: 10.1016/J.VACCINE.2009.09.008 [retrieved on 2009-09-15]
- SANCHEZ J ET AL: "Virulence factors, pathogenesis and vaccine protection in cholera and ETEC diarrhea", CURRENT OPINION IN IMMUNOLOGY, ELSEVIER, OXFORD, GB, vol. 17, no. 4, 1 August 2005 (2005-08-01) , pages 388-398, XP027787152, ISSN: 0952-7915 [retrieved on 2005-08-01]
- HOLMGREN J ET AL: "An oral B subunit: whole cell vaccine against cholera", VACCINE, ELSEVIER LTD, GB, vol. 10, no. 13, 1 January 1992 (1992-01-01), pages 911-914, XP023710802, ISSN: 0264-410X, DOI: 10.1016/0264-410X(92)90324-D [retrieved on 1992-01-01]
- SHAMSUZZAMAN S ET AL: "Robust gut associated vaccine-specific antibody-secreting cell responses are detected at the mucosal surface of Bangladeshi subjects after immunization with an oral killed bivalent V. cholerae O1/O139 whole cell cholera vaccine: Comparison with other mucosal and systemic responses", VACCINE, ELSEVIER LTD, GB, vol. 27, no. 9, 25 February 2009 (2009-02-25), pages 1386-1392, XP026194432, ISSN: 0264-410X, DOI: 10.1016/J.VACCINE.2008.12.041 [retrieved on 2009-01-13]
- ANH ET AL: "Safety and immunogenicity of a reformulated Vietnamese bivalent killed, whole-cell, oral cholera vaccine in adults", VACCINE, ELSEVIER LTD, GB, vol. 25, no. 6, 20 December 2006 (2006-12-20), pages 1149-1155, XP005809564, ISSN: 0264-410X, DOI: 10.1016/J.VACCINE.2006.09.049
- HOLMGREN J ET AL: "New cholera vaccines", VACCINE, ELSEVIER LTD, GB, vol. 7, no. 2, 1 April 1989 (1989-04-01), pages 94-96, XP023798738, ISSN: 0264-410X, DOI: 10.1016/0264-410X(89)90042-X [retrieved on 1989-04-01]
- CLEMENS J D ET AL: "Field trial of oral cholera vaccines in Bangladesh: evaluation of anti-bacterial and anti-toxic breast-milk immunity in response to ingestion of the vaccines", VACCINE, ELSEVIER LTD, GB, vol. 8, no. 5, 1 October 1990 (1990-10-01) , pages 469-472, XP023712189, ISSN: 0264-410X, DOI: 10.1016/0264-410X(90)90248-K [retrieved on 1990-10-01]
- JERTBORN M ET AL: "Safety and immunogenicity of an oral recombinant cholera B subunit-whole cell vaccine in Swedish volunteers", VACCINE, ELSEVIER LTD, GB, vol. 10, no. 2, 1 January 1992 (1992-01-01), pages 130-132, XP023710419, ISSN: 0264-410X, DOI: 10.1016/0264-410X(92)90030-N [retrieved on 1992-01-01]
- LYCKE N ET AL: "Strong biotype and serotype cross-protective antibacterial and antitoxic immunity in rabbits after cholera infection", MICROBIAL PATHOGENESIS, ACADEMIC PRESS LIMITED, NEW YORK, NY, US, vol. 1, no. 4, 1 August 1986 (1986-08-01), pages 361-371, XP023313147, ISSN: 0882-4010, DOI: 10.1016/0882-4010(86)90068-9 [retrieved on 1986-08-01]
- TAYLOR ET AL: "Evaluation of a bivalent (CVD 103-HgR/CVD 111) live oral cholera vaccine in adult volunteers from the United States and Peru.", INFECTION AND IMMUNITY, vol. 65, no. 9, 1 September 1997 (1997-09-01), pages 3852-3856, XP055048355, ISSN: 0019-9567
- TAYLOR ET AL: "Expanded safety and immunogenicity of a bivalent, oral, attenuated cholera vaccine, CVD 103-HgR plus CVD 111, in United States military personnel stationed in Panama.", INFECTION AND IMMUNITY, vol. 67, no. 4, 1 April 1999 (1999-04-01), pages 2030-2034, XP055048356, ISSN: 0019-9567
- MANNING ET AL: "Molecular cloning and expression in Escherichia coli K-12 of the O antigens of the Inaba and Ogawa serotypes of the Vibrio cholerae O1 lipopolysaccharides and their potential for vaccine development.", INFECTION AND IMMUNITY, vol. 53, no. 2, 1 August 1986 (1986-08-01) , pages 272-277, XP055048384, ISSN: 0019-9567
- MICHAEL LEBENS ET AL: "Construction of novel vaccine strains ofco-expressing the Inaba and Ogawa serotype antigens", VACCINE, vol. 29, no. 43, 30 July 2011 (2011-07-30) , pages 7505-7513, XP028306514, ISSN: 0264-410X, DOI: 10.1016/J.VACCINE.2011.06.121 [retrieved on 2011-07-20]
- CHIANG ET AL: 'Construction of a Vibrio cholerae vaccine candidate using transposon delivery and FLP recombinase- mediated excision' INFECTION AND IMMUNITY vol. 68, no. 11, 2000, pages 6391 - 6397, XP008153886
- 'PRODUCT MONOGRAPH, DUKORAL(R), ATCC Code: J07AE01', [Online] 2007, XP008159292 Retrieved from the Internet: <URL:https://www.vaccineshoppecanada.com/se cure/pdfs/ca/dukoral_e.pdf>
- DUTTA ET AL: 'Spread of cholera with newer clones of Vibrio cholerae O1 El Tor, serotype inaba, in India' JOURNAL OF CLINICAL MICROBIOLOGY vol. 44, no. 9, 2006, pages 3391 - 3393, XP008153884 & DATABASE UNIPROT [Online] 04 April 2006 EBI Database accession no. Q27HR8
- TOBIAS ET AL: 'Construction of non-toxic Escherichia coli and Vibrio cholerae strains expressing high and immunogenic levels of enterotoxigenic E. coli colonization factor I fimbriae' VACCINE vol. 26, no. 6, 2008, pages 743 - 752, XP022425413
- STROEHER ET AL: 'Serotype conversion in Vibrio cholerae O1' PNAS vol. 89, no. 7, 1992, pages 2566 - 2570, XP008153868

## Description

### Technical field the invention

The present invention relates to the field of vaccines, in particular vaccines against cholera and enterotoxigenic *E. coli* (ETEC) diarrhea.

### Background to the invention

Cholera remains a major health problem in large parts of the world. This is also true for ETEC, which is the main cause of diarrheal disease in developing countries as well as in travelers to these countries. In many developing countries effective water and sanitary measures for control of cholera and other enteric infections are currently impossible, and in this context, vaccines have an important role to play. In order to do so however, they need to be effective, readily accessible and above all cheap. There is also a medical need and a very substantial commercial market for use of cholera and especially ETEC vaccines in travelers.

One approach has been the development of oral killed whole cell vaccines. Dukoral ™ is an oral cell vaccine (OCV) with demonstrated up to 90% efficacy against cholera and also a significant efficacy against Enterotoxigenic *Escherichia coli* (ETEC)-induced diarrhea. It comprises 3 different *V. cholerae* strains in four different formulations (two heat-killed and two formalin-killed) and in addition recombinantly produced cholera toxin B subunit (rCTB). The rCTB component contributes significantly to the efficacy against cholera and is solely responsible for the observed protection against ETEC diarrhea due to its ability to induce cross-neutralizing antibodies against the cholera toxin (CT)-like *E. coli* heat-labile toxin (LT). However, rCTB is acid-labile and thus the vaccine (which needs to be given in two doses) must be administered with a bicarbonate buffer.

Despite Dukoral™ being the only internationally licensed OCV, copies of this vaccine with or without the CTB component are currently being marketed in developing countries - Vietnam, India and China. The OCV made in Vietnam and India (which lacks the CTB component) contains the same 4 bacterial components as in Dukoral plus a fifth formalin-killed *V. cholerae* strain of serogroup 0139.

Protective immunity against cholera elicited by OCVs is mainly if not exclusively based on mucosal production of antibodies against cell wall lipopolysaccharide O1 (O1 LPS) and for the CTB-containing Dukoral vaccine also antitoxin antibodies in the intestine.

From the above it is evident that the present state of the art for production of cholera/ETEC vaccine is far from simple, and although already effective, a real contribution to making a cholera vaccine more accessible would be to rationalize the composition of the formulation at several levels.

The necessity to include several different *Vibrio cholerae* strains in killed whole cell vaccines such as Dukoral™ arises from the need to represent several different antigenic variants of *Vibrio cholerae* in the vaccine. All protective strains in the currently used vaccines are of the O1 serogroup which until 1993 was the only one of more than 200 identified serogroups known to cause epidemic cholera and is still the dominant serogroup. However, the O1 serogroup has two variants called the Ogawa and Inaba serotypes that differ in the methylation of the terminal sugar of the O-antigen of the surface lipopolysaccharide (LPS). Serotype switching is known to occur in which the Ogawa serotype organism can give rise to Inaba organisms. The reverse switch however is rare.

Although immunization with especially Inaba but also Ogawa serotype can give rise to antibodies cross-reacting with the other serotypes it also gives rise to serotype specific antibodies that contribute significantly to protection. Thus, an effective vaccine should induce not only cross reactive but also serotype-specific antibodies against both Inaba and Ogawa serotype variants.

The serotype switch is known to be related to a mutation in a single gene (*wbeT*)*.* Any mutation that inactivates this gene results in a switch from the Ogawa to the Inaba serotype. Mutations that can reverse such an event are predictably much more uncommon although a switch from the Inaba to the Ogawa serotype can easily be achieved by provision of the relevant gene *in trans.* The gene involved (*wbeT,* also denoted *rfbT*) encodes a methyl transferase that methylates the terminal perosamine residue in the O-antigen polysaccharide repeating unit. Mutations in this gene that lead to the Inaba serotype are almost invariably insertions, deletions or base changes that introduce a nonsense codon.

A third O1 variant known as Hikojima has also been documented to occur in the wild. Hikojima is characterized by that it expresses both the Ogawa and Inaba determinants on its surface and agglutinates with antisera specific for both types. The Hikojima phenotype is extremely rare and is considered in the literature to be an unstable transitional form.

Chiang and Mekalonos (Infection and Immunity, vol. 68, no. 11, 1 January 2000 (2000-01-01), pages 6391-6397, XP008153886, ISSN: 0019-9567, DOI: 10.1128/1A1.68.11) report construction of a *Vibrio cholerae* vaccine candidate using transposon delivery and FLP recombinase mediate excision. A motile Inaba+ vaccine candidate, Peru-2, was converted into nonmotile Ogawa+ by the manipulation.

Rijpkema et al. (Journal of Medical Microbiology, vol. 53, no. 11, 1 November 2004 (2004-11-01), pages 1105-1107, XP055048436, ISSN: 0022-2615, DOI: 10.1099/jmm.0.45744-0) report analysis of *WbeT* mutations in clinical *Vibrio cholerae* isolates.

Kanungo et al. (Vaccine, vol. 27, no. 49, 16 November 2009 (2009-11-16), pages 6887-6893, XP026722082, ISSN: 0264-410X, DOI: 10.1016/J.Vaccine.2009.09.008) report a randomized placebo-controlled trial of a reformulated, bivalent, killed, whole-cell, oral cholera vaccine conducted among adults and children in Kolkata, India.

Sanchez and Holmgren (Current Opinion in Immunology, vol. 17, no. 4, 1 August 2005 (2005-08-01), pages 388-398, XP027787152, ISSN: 0952-7915) review virulence factors, pathogenesis and vaccine protection in cholera and ETEC diarrhea.

With this in mind the inventors have set out to engineer a single vaccine strain of *V. cholerae* that would effectively replace the three currently used strains.

Thus, it is an object of the invention to provide an efficient vaccine against cholera and/or ETEC diarrhea, with simplified formulation and with lower productions costs and that also ideally produces protective immunity after single administration.

### Summary of the invention

The present invention describes the construction, method of manufacture, formulation and medical-preventive use of a novel cholera and/or ETEC vaccine. Not all aspects disclosed herein are part of the presently claimed invention. The claimed subject matter is limited to that specified in the appended claims.

Throughout this text, in line with established scientific practice, the designation *"wbeT"* (in italics) denotes the **gene,** whereas the designation *"WbeT"* (in italics) denotes a **protein** coded for by a *wbeT* gene.

In a **first aspect,** a vaccine comprising a *Vibrio cholerae* O1 cell, characterized in that said cell comprises O1 antigens of both Ogawa and Inaba serotypes is provided, wherein the vaccine comprises multiple *Vibrio cholerae* O1 cells that comprise O1 antigens of both Ogawa and Inaba serotypes, and wherein on average, 10-90 % of the O1 antigens of said cells are of the Ogawa-serotype.

More preferably, 10-70 % of the O1 antigen expressed by the cells is of the Ogawa-serotype. Yet more preferably, 10-50 % of the O1 antigen expressed by the cells is of the Ogawa-serotype. Still more preferably, 10-40 % of the O1 antigen expressed by the cells is of the Ogawa-serotype. Most preferably, 10-30 % of the O1 antigen expressed by the cells is of the Ogawa-serotype.

The cell of the vaccine may further comprise one or more ETEC colonization factor (CF) protein(s), such as CFA/I, CS2 or CS5, wherein said CF protein(s) is/are expressed either as single, double or hybrid fimbriae.

Preferably, said vaccine does not contain any further immunogically active whole cells in addition to *Vibrio cholerae* O1 cells that comprise O1 antigens of both Ogawa and Inaba serotypes.

Preferably, the vaccine is for oral administration. Preferably, the cell in the vaccine is formalin-inactivated.

Preferably, the cell is a genetically modified cell, preferably a genetically modified cell according the seventh or eighth aspects of the invention (see below).

In a **second aspect** a vaccine according to the first aspect is provided, for use in preventive immunization, preferably for use in preventive immunization against cholera and/or enterotoxigenic *Escherichia coli*-infection (ETEC).

In a **third aspect** (not part of the claimed invention), a method for inducing preventive immunity is described, comprising administering a vaccine according to the first aspect to a subject to be immunized. Preferably, the preventive immunity is against cholera and/or enterotoxigenic *Escherichia coli*-infection (ETEC). Also preferably, the the administration is done orally.

In a **fourth aspect** (not part of the claimed invention as such), a DNA-construct is disclosed, comprising DNA coding for a *WbeT*-protein having at least 70 % sequence identity to SEQ ID NO: 6 (more preferably at least 80 % identity, even more preferably at least 90 % identity, yet more preferably at least 95 % identity and most preferably at least 99 % identity) operatively coupled to a promoter suitable for inducing protein expression in a *Vibrio cholerae* O1 host cell, characterized in that the coded *WbeT-*protein comprises sequence modifications in relation to SEQ ID NO: 6 that reduce the enzymatic activity of the coded protein relative to the enzymatic activity of a protein with a sequence identical to SEQ ID NO: 6.

Preferably, the sequence modifications comprise a substitution of the serine residue in position 158 of SEQ ID NO: 6, more preferably a substitution of the serine residue in position 158 of SEQ ID NO: 6 to glycine, proline, threonine, phenylalanine or tryptophan.

In a **fifth aspect** (not part of the claimed invention as such), a DNA-construct is disclosed, comprising DNA coding for a *WbeT*-protein having at least 70 % sequence identity to SEQ ID NO: 6 (more preferably at least 80 % identity, even more preferably at least 90 % identity, yet more preferably at least 95 % identity and most preferably at least 99 % identity) operatively coupled to a promoter suitable for inducing protein expression in a *Vibrio cholerae* O1 host cell, characterized in that the promoter is suitable for inducing the expression of the coded *WbeT*-protein in a *Vibrio cholerae* O1 host cell initially of Inaba phenotype (i.e. host cell which is Inaba prior to transformation by the DNA construct) to such level of transgenic *WbeT* protein expression as to allow simultaneous expression of both Inaba and Ogawa antigens by the host cell.

Preferably, the promoter of the above aspects is an inducible promoter, such as a *tac* or *lac* promoter.

Preferably, the DNA construct of the above aspects is a plasmid vector capable of replication in a host cell or a vector capable of chromosomal integration in a host cell.

Preferably, the DNA-construct according to the above aspects further comprises a selectable marker, more preferably a positive selectable marker such as antibiotic resistance gene or a metabolic selectable marker.

In a **sixth aspect** (not part of the claimed invention as such), a DNA construct for homologous recombination in a Vibrio Cholerae O1 host is described, characterized in that the construct is adapted to modifying the endogenous *wbeT* gene of the host by means of homologous recombination. Preferably, the DNA-construct according to the sixth aspect further comprises a selectable marker, more preferably a positive selectable marker such as antibiotic resistance gene or a metabolic selectable marker.

In a **seventh aspect** (not part of the claimed invention as such), a *Vibrio cholerae* O1 cell simultaneously expressing both Inaba and Ogawa antigens is disclosed, characterized in that
a. the endogenous *wbeT*-gene of the host cell or the protein coded thereof is inactive;
b. the cell comprises a recombinant DNA-construct inducing expression of WbeT enzyme activity; and wherein
c. the level of transgenic *WbeT* enzyme activity is such that the cell simultaneously expresses Inaba and Ogawa antigens.

Preferably, the recombinant DNA-construct of the above aspects is a DNA construct according to the fourth to fifth aspects.

In an **eighth aspect,** a *Vibrio cholerae* O1 cell simultaneously expressing both Inaba and Ogawa antigens is provided, characterized in that
a. the cell comprises an endogenous *wbeT*-gene; and
b. the cell comprises a recombinant DNA-construct capable of modulating the expression level of endogenous *wbeT* gene or the enzymatic activity of the product thereof; and wherein
c. the modulated level of *WbeT* enzyme activity is such that the cell simultaneously expresses Inaba and Ogawa antigens, wherein 10-90 % of the O1 antigen expressed by the cell is of the Ogawa-serotype.

Preferably, the recombinant DNA-construct of the above aspect is a DNA construct according to the sixth aspect.

Preferably, 10-90 % of the O1 antigen expressed by the cell of the above aspects is of the Ogawa-serotype. More preferably, 10-70 % of the O1 antigen expressed by the cell of the above aspects is of the Ogawa-serotype. Yet more preferably, 10-50 % of the O1 antigen expressed by the cell of the above aspects is of the Ogawa-serotype. Still more preferably, 10-40 % of the O1 antigen expressed by the cell of the above aspects is of the Ogawa-serotype. Most preferably, 10-30 % of the O1 antigen expressed by the cell of the above aspects is of the Ogawa-serotype.

Preferably, the cell of the above aspects further expresses one or more ETEC colonization factor (CF) protein(s), such as CFA/I, CS2 or CS5, wherein said CF protein(s) is/are expressed either as single, double or hybrid fimbriae.

In a **ninth aspect,** a method for manufacturing a vaccine is provided, comprising the steps of:
providing a *Vibrio cholerae* O1 cell comprising O1 antigens of both Ogawa and Inaba serotypes; and
killing said cell.

Preferably, the killing is performed by formalin-treatment or by heat-treatment.

Preferably, the cell is a cell according to the seventh or the eighth aspects.

In a **tenth aspect** (not part of the claimed invention), the present disclosure also describes a kit for use in vaccination formulated as a one-unit composition, whereby the composition is presented in one part of the kit and instructions for use in another part.

### Detailed description of the invention

### Vaccine comprising cells with O1 antigens of both Ogawa and Inaba serotypes

Although *V. cholerae* of serogroup 0139 can also cause cholera, >98% of all cholera worldwide is caused by *V. cholerae* O1. The O1 serogroup has two subtypes/serotypes - Ogawa and Inaba. Serotype switching from the Ogawa to the Inaba subtype occurs at a relatively high frequency whereas the reciprocal conversion is rare. The basis of the serotype switch is a mutation in the LPS synthetic pathway that leads to change in the structure of the O1 antigen. An effective vaccine need to include both Ogawa and Inaba strains in its composition since their LPS are serologically distinct with both shared and distinct epitopes contributing to protection.

A vaccine comprising *Vibrio cholerae* O1 cells expressing O1 antigens of both Ogawa and Inaba serotypes is disclosed, and has the advantage of simplifying production because it obviates the need to use distinct cells for each of the phenotypes in the manufacture of the vaccine. By enabling the use of single type of cell, the manufacture of the vaccine is also simplified, since only one type of inactivation treatment is needed.

Immunization with vaccine of the invention based on single *V. cholerae* strain that express different amounts of both Ogawa and Inaba serotypes gives rise to cross-reactive as well as type-specific antibodies to both Ogawa and Inaba antigens (see example 1).

On average, 10-90 % of the O1 antigens of the cells are of the Ogawa-serotype. The Inaba-antigen is preferably present in a higher amount (meaning more than 50 %) than the Ogawa-antigen, as the Inaba-antigen can elicit a certain level of cross-serotype protection against Ogawa, whereas Ogawa-antigen can only elicit protection against itself.

The efficacy of the vaccine against ETEC may further be improved by incorporation of the feature that the cells further express one or more ETEC colonization factor (CF) protein(s), such as CFA/I, CS2 or CS5, wherein said CF protein(s) is/are expressed either as single, double or hydrid fimbriae (see below for details).

It is preferable, that the above vaccine does not contain any further immunogically active whole cells in addition to *Vibrio cholerae* O1 cells that comprise O1 antigens of both Ogawa and Inaba serotypes. However, the above vaccine may further comprise recombinant CTB in a manner similar to Dukoral™.

The vaccine is preferably for oral administration, but may also be administered by injection. Preferably, the vaccine comprises *Vibrio cholerae* O1 cells that comprise O1 antigens of both Ogawa and Inaba serotypes that are formalin-inactivated.

Preferably, the vaccine comprises genetically modified *Vibrio cholerae* O1 cells, preferably such genetically modified *Vibrio cholerae* O1 cells as described below.

The vaccine of the invention may be a vaccine composition comprising one or more pharmaceutically acceptable, excipients, carriers, diluents and adjuvants.

The formulation of vaccine compositions according to the invention is well known to persons skilled in the art. Suitable pharmaceutically acceptable carriers and/or diluents include any and all conventional solvents, dispersion media, fillers, solid carriers, aqueous solutions, coatings, antibacterial and antifungal agents, isotonic and absorption delaying agents, and the like. The use of such media and agents for pharmaceutically active substances is well known in the art, and is described, by way of example, in Remington's Pharmaceutical Sciences, 18th Edition, Mack Publishing Company, Pennsylvania, USA. Except insofar as any conventional media or agent is incompatible with the active ingredient, use thereof in the pharmaceutical compositions of the present invention is contemplated. Supplementary active ingredients can also be incorporated into the compositions.

The vaccine composition for oral use may preferably comprise 10⁸-10¹⁴ cells/ml, more preferably 10¹⁰-10¹² cell per ml, and most preferably about 10¹¹ cells/ml.

The vaccine composition for oral use may be formulated as a foodstuff, beverage or a feed supplement (when for use in immunizing animals).

The vaccine composition may comprise an adjuvant known in the art, or may lack any adjuvants.

### Use of the vaccine

The present invention discloses use of the above vaccine in preventive immunization, preferably against cholera and/or enterotoxic *Escherichia coli*-infection (ETEC).

Preferably, the vaccine is administered orally or sublingually.

The administration may also be done by injection.

The vaccine is preferably used to immunize humans and other mammals, such as pets (cats, dogs and the like) or farm animals (such as cows, horses, sheep, goats, pigs and the like).

Preferably, the vaccine is administered orally at 10⁸-10¹⁴ cells per dose, more preferably 10¹⁰-10¹² cells per dose, and most preferably about 10¹¹ cells per dose.

The immunization protocol may consist of a single administration or may comprise two or more administrations. In a preferred embodiment, the initial immunization protocol to induce protective immunity comprises a first administration and a second administration, separated in time by at least 7 days but by no more than about 2 months. After the initial immunization protocol, protective immunity may be maintained as long as desired by booster administrations occurring with less than 3 year intervals, preferably less than 2 year intervals. It is may be preferable that a booster administration does not take place before at least 1 year has elapsed from the first administration.

### Method of manufacture for a vaccine

A method for manufacturing a vaccine is disclosed, comprising the steps of:
a. providing a Vibrio cholerae O1 cell comprising O1 antigens of both Ogawa and Inaba serotypes; and
b. killing said cell, such as by formalin treatment or by heat treatment.

Preferably, the killing is performed by formalin-treatment. Preferably the cell is a genetically modified cell, preferably such as described below.

Besides having the advantage enabling the use of a single inactivation (killing) method, the vaccine may be manufactured using standard protocols known e.g. from the manufacture of Dukoral™.

### Genetically modified cells useful for vaccine manufacture and DNA constructs for obtaining such cells

The *Vibrio cholerae* cells comprising O1 antigens of both Ogawa and Inaba serotypes comprised in the vaccine could in principle be obtained from a naturally occurring strain having a Hikojima phenotype. However, to the best knowledge of the inventors such strains are very rare and no such strains are presently available to the public. In the literature, such natural strains have also been described as unstable, which renders them less promising for industrial production of vaccines.

Thus, the inventors have derived *V. cholerae* cells that express O1 antigens of both Ogawa and Inaba serotypes by way of genetic engineering and have obtained novel strains with stable Hikojima phenotype. Cells derived in this manner also have the advantage that any desired strain (such as a known and well-characterized vaccine strain) can be used as a starting point, substantially simplifying the production and streamlining the experiments needed for GMP production and regulatory approval.

The inventors demonstrate herein that the key parameter for obtaining the desired Hikojima phenotype is to obtain a suitable level of *WbeT* enzyme activity. By suitable in this context is meant that the *WbeT* enzyme activity level of the cell is not so low that the cells have an essentially pure Inaba phenotype and not so high that the cells have an essentially pure Ogawa phenotype.

In the context of the present invention, 10-90% of the O1 antigens on the cells are of the Ogawa type (with remainder consequently of the Inaba type). More preferably, 10-80% of the O1 antigens on the cells are of the Ogawa type, yet more preferably 10-50%, still more preferably 10-40% and most preferably 20-30%.

As shown in the Examples below, a suitable Hikojima phenotype as outlined above is obtainable by several distinct strategies utilizing recombinant DNA technology:
a) A mutant *WbeT* protein having low enzymatic activity may be expressed at high levels in a host having Inaba phenotype;
b) A *WbeT* protein having high enzymatic activity may be expressed at low levels in an Inaba host; or
c) The endogenous *WbeT* gene of an Ogawa host may be mutated e.g. by means of homologous recombination to render the resulting protein to have suitably reduced activity.
d) The endogenous *WbeT* gene of an Inaba host may be replaced or modified e.g. by means of homologous recombination to render the protein produced by the gene to have suitably increased activity.

The present disclosure discloses cells obtained by each of the above strategies, as well as DNA-constructs suitable for obtaining cells by each of the above strategies. However, as specified in the appended claims, only certain embodiments of cells are part of the presently claimed invention as such.

From the teachings herein it is apparent to the skilled person that achieving the desired level of *WbeT* expression by the strategies outlined above can be realized in many different ways. For instance, levels of expression of a *WbeT* transgene may be modulated by using an inducible promoter (such as *cat, lac* or *tac*) whereby the level of expression may be modulated during culture of the host cells by adjusting the level of the inducer that the host cells are exposed to.

Alternatively, several weak and strong constitutional promoters are also known and may be used in conjunction with a suitably modified *WbeT* protein. A weak promoter can be used to constitutionally induce a very low level of expression of a highly active (such as wild-type; SEQ ID NO: 6) *WbeT* protein. Conversely, a strong constitutional promoter can be used to induce a high level of expression of a *WbeT* protein having low activity (such as a mutated *WbeT* protein, preferably such as described below).

Both plasmids and chromosomally integrated *wbeT* transgenes may be used in the cells to achieve the desired phenotype.

Many different mutations of the *WbeT*-protein can potentially result in a suitably active protein, and such mutated variants can readily be obtained by the skilled person using methods well known in the arts by mere routine experimentation, on the basis of the teachings herein. Whether a cell of the desired phenotype is obtained by expressing the mutated *WbeT* protein or not can readily be analyzed by the skilled person e.g. using the methods disclosed in Example 5. The inventors have identified serine 158 in the *WbeT* protein (SEQ ID NO: 6) as a suitable residue to modulate activity. Thus, the mutations preferably comprise a substitution on serine 158, more preferably substitution of serine 158 to glycine, proline, valine, leucine, alanine, threonine, methionine, tryptophan, arginine or phenylalanine. Most preferably, the serine 158 is substituted by glycine, proline, threonine, phenylalanine or tryptophan.

### ETEC colonization factor (CF) protein(s)

As is evident from the above, the vaccine of the invention (or rather the cells on which the vaccine is based upon) may also comprise other enhanced features besides the combined expression of O1 antigens of both Inaba and Ogawa serotypes. In particular, the cells may express one or more ETEC colonization factor (CF) proteins, such as CFA/I, CS2 or CS5, wherein said CF protein(s) is/are expressed either as single, double, or hybrid fimbriae, as demonstrated in Example 7. Inclusion of such CF proteins in the cells of the vaccine is especially useful for inducing protective immunity against ETEC.

The expression "comprising" as used herein should be understood to include, but not be limited to, the stated items.

The invention is further illustrated by the following examples, which are to be seen as non-limiting.

### Example 1. Preparation and testing of a vaccine comprising Vibrio cholerae cells comprising O1 antigens of both Ogawa and Inaba serotypes

It was tested whether a vaccine comprising *V. cholerae* bacteria of strain JS1569 (Inaba) that had been genetically modified to express O1 antigens of both Ogawa and Inaba serotypes would give rise to an antibody response with a different proportion of antibodies reacting with Ogawa and Inaba LPS in ELISA as compared to the antibody response after immunization with the parent JS1569 Inaba strain.

The bacteria from example 2 (see below) were formalin-killed and used for immunization. Formalin-killing of bacteria and oral immunizations and assay methods were performed as previously described (Nygren E, Li BL, Holmgren J, Attridge SR. Infect Immun. 2009 Aug;77(8):3475-84). Briefly, Balb/c mice were immunized in 3 rounds at 2-week intervals with two daily doses of 3x10⁸ formalin-killed cells (together with an adjuvant for the WbeT strain), and one week after the last immunization the mice were sacrificed and serum collected and tested for combined IgG/IgM antibody titers in ELISA plates coated with either Inaba or Ogawa LPS.

The results are presented in the table below and show that in contrast to the parent JS1569 Inaba strain which gave rise to an antibody response with a slightly higher anti-Inaba than anti-Ogawa titer, the JS1569/wild type Wbe S158S vaccine gave rise to an antibody response with a much higher anti-Ogawa than anti-Inaba titer, although it still gave rise also to modest formation of specific anti-Inaba antibodies:

| **Immune serum** | **Inaba/Ogawa titers (Ratio)** |
|---|---|
| To JS1569 | 10290/5060 (2:1) |
| Same absorbed with Ogawa | 2940/160 (18:1) |
| To JS1569/ wild type Wbe S158S | 36000/365000 (1:10) |
| Same absorbed with Inaba | 1600/49000 (1:30) |
| Same absorbed with Ogawa | 1700/2500 (1:1.5) |

These findings were confirmed when immunizations were given subcutaneously without adjuvant. In marked difference to immunization with the parent JS1569 Inaba strain and more similar to immunization with the Ogawa A457 reference strain, immunization with JS1569 WbeT gave rise to immune serum with a strong proportion of Ogawa specific antibodies, as shown in the table below.

| **Immune serum** | **Inaba titer** | **Ogawa titer** | **Ratio Inaba/Ogawa** |
|---|---|---|---|
| JS1569 Inaba | 480 | 260 | 1.8:1 |
| JS1569 abs with Ogawa A457 | 180 | 60 | 3:1 |
| JS1569 abs with 1569 WbeT | 240 | 60 | 4:1 |
| A457 Ogawa | 660 | 1940 | 1:1.9 |
| A457 abs with Inaba 1569 | 240 | 1020 | 1:4.3 |
| A457 abs with JS1569 WbeT | 180 | 40 | 1.8:1 |
| JS1569 WbeT | 420 | 1580 | 1:3.8 |
| WbeT abs with Ogawa A457 | 150 | 60 | 2.5:1 |
| WbeT abs with Inaba JS1569 | 180 | 920 | 1:5.1 |
| WbeT abs with JS1569 WbeT | 120 | 100 | 1.2:1 |

### Example 2: Genetically modified Vibrio cholerae cells expressing O1 antigens of both Ogawa and Inaba serotypes by expression of mutated WbeT-protein: Plasmid-based expression of mutated WbeT-protein

In a single entry in GenBank of the *wbeT* gene from a Hikojima strain there is a mutation converting a serine to proline at position 158 of the protein (S158P) although the same mutation has been described in a strain identified as being of the Inaba serotype (GenBank accession numbers FJ619106 and DQ401028 respectively). Having amplified the wild-type *wbeT* gene from the O1 El Tor Ogawa strain VX44945 with primers wbeT1 EcoRI (SEQ ID NO: 1 5'-CCCGGTCTCGAATTC CTGCATCTGCAAGTTGATTCTGTATG-3') and wbeT2 HindIII (SEQ ID NO: 2 5'-CCCGGTCTCAAGCTTATAGTGAACTCTTCGGAAATGTCTG-3'), it was digested with Eco31I and cloned into an expression vector derived from pAF1 () in which the cloned gene was placed under the control of the powerful synthetic tac promoter that had been digested with EcoRI and HindIII. The sequence of the gene was confirmed by DNA sequencing of the plasmid with primers wbe1 (SEQ ID NO: 3 5'-CTGCATCTGCAAGTTGATTCTGTATG-3') and wbe2 (SEQ ID NO: 4 5'-ATAGTGAACTCTTCGGAAATGTCTG-3').

The DNA sequence of the wild-type *wbeT* gene is shown in SEQ ID NO: 5 whereas the wild-type protein is shown in SEQ ID NO: 6.

The full sequence of the plasmid (pML-wbeTtac) expressing the wild-type *wbeT* is shown in SEQ ID NO: 7.

In order to construct the mutant library of *wbeT* carrying mutations at amino acid position 158 of the gene product, oligonucleotides wbeT m3 (SEQ ID NO: 8 5'-GCGCGCCAGAACTTGGCTATTTTTAACC-3') and wbeT m1 (SEQ ID NO: 9 5'-GGGGGTTCGAAGTTTATGAGTTTGATAATAGGGTGNNBTCATTATATTTTCAAAAAAATACA GACATAGCAGATAAGGTTAAAAATAGCCAAGTTCTGGCGCGC-3') were synthesized. The two oligonucleotides were mixed in equimolar quantities and allowed to anneal at room temperature overnight. Full length double-stranded DNA was made by extension of the short wbeT m3 primer using T4 DNA polymerase in the presence of excess deoxyribonucleotide triphosphates. The resulting fragment was digested with Bsp119I and Van91I and ligated into pML*-wbeTtac* (SEQ ID NO: 7) digested with the same enzymes. The ligated DNA was used to transform commercially obtained electro-competent *E. coli* strain DH12S (Invitrogen). After incubation without antibiotic selection a small aliquot of the cells were spread onto a selective LB agar plate supplemented with ampicillin (100 µg/ml). The rest of the cells were diluted to 25 ml with fresh LB broth. Ampicillin was added to a final concentration of 100 µg/ml and the culture was grown overnight at 37° C in order to obtain a clone library.

Aliquots of the resulting culture were supplemented with glycerol to a final concentration of 17% and stored at -70° C. Other aliquots were used to prepare plasmid DNA.

The colonies obtained on the LB agar plate were picked onto a fresh plate and colonies were cultured to prepare plasmid DNA. The plasmids were sequenced in order to determine whether the *wbeT* genes carried mutations. Mutants of *wbeT* obtained from the library are the following: S158G, S158P, S158V, S158I, S158L, S158A, S158T, S158M, S158W, S158R, S158C and S158F. Additionally the wild-type gene and a gene with the stop signal TGA at position 158 were isolated.

The different plasmids were isolated and used to transform the O1 classical Inaba strain JS1569. This strain has a mutant *wbeT* gene with the glycine (GGA) at position 219 of the protein being changed to a stop codon (TGA) resulting in a truncated and inactive product (SEQ ID NO: 10 and SEQ ID NO: 11).

There are other polymorphisms that do not appear to have any significance.

The different strains generated by the introduction of the different recombinant plasmids expressed different levels of the Ogawa antigen when grown under inducing conditions (in the presence of 1mM IPTG). The phenotype was assessed on the basis of agglutination assays and in some cases using inhibition ELISA (see Example 5 for description of materials and methods). The wild type gene gave rise to almost total serotype switching whereas others (such as S158P and S158G) gave slight but detectable agglutination with Ogawa-specific antiserum as well as agglutination with an Inaba-specific antiserum (and therefore conferred a Hikojima serotype). Some mutants had no detectable activity with Ogawa-specific antiserum (S158I and S158C) and yet others gave intermediate agglutination (S158T, S158F and S158W).

The results demonstrate unambiguously that mutations and specifically mutations at position 158 of the *wbeT* gene product result in proteins with altered enzyme activity. At present there is no reliable assay to directly quantitatively determine the levels of enzyme activity of these mutants compared to the wild-type, but the relevant end result can readily be evaluated as in Example 5. In summary, all except S158C and S158I were able to complement the Inaba phenotype of the host strain to some extent.

### Example 3: Genetically modified Vibrio cholerae cells expressing O1 antigens of both Ogawa and Inaba serotypes by expression of mutated WbeT-protein: Chromosomal insertion of mutant wbeT

The truncated chromosomal *wbeT* gene in the strain JS1569 was substituted with the mutant genes generated in example 2. The relevant mutated genes were amplified with primers wbeT1 BlgII (SEQ ID NO: 1) and wbeT2 BglII (SEQ ID NO: 2). Amplified fragments were digested with BglII and ligated into the suicide vector pMT-SUICIDE (SEQ ID NO: 12) which had been digested with BamHI. This is a small R6K-based suicide vector constructed in this laboratory by M. Lebens which carries the chloramphenicol resistance gene and the origin of transfer (oriT) from the broad host-range plasmid RP4 that allows the plasmid to be conjugally transferred to V. cholerae strain with the aid of a helper plasmid (pNJ5000; Grinter NJ, Gene. 1983 JanFeb;21(1-2):133-43).

In the clones that were generated, the *wbeT* genes (S158G and the wild-type) were both inserted with the cloned genes in the opposite orientation to the *cat* gene. The sequence of such vector is exemplified by SEQ ID NO: 13 (carrying wild-type *wbeT* gene; the construct for S158G is identical save for the nucleotides coding for *WbeT* residue 158).

The resulting plasmids were mated into strain JS1569 and selected on the basis of chloramphenicol and rifampicin resistance. Since the plasmid has no counter-selection for loss of the plasmid, its insertion into the chromosome by homologous recombination results in tandem copies of the *wbeT* gene separated by the plasmid. Depending upon where the recombination occurred, the clones had different phenotypes (see Example 5).

The strain that had received the wild-type gene (1342) had a clear Hikojima phenotype. Inhibition ELISA showed that it expressed only 15% of the Ogawa LPS present on the surface of the strain that that received the S158G mutant. The latter strain (1356) was in effect an Ogawa strain that agglutinated strongly with Ogawa-specific antiserum, but not at all with the Inaba-specific antiserum.

The strains were however very stable; they retained their LPS serotypes and remained chloramphenicol resistant even in the absence of selection, indicating that the plasmid was not readily lost.

PCR and sequencing using the wbe1 and 2 primers (SEQ ID NO: 3 and 4, respectively) showed that there were two genes in the strains that varied at sites of variation between the gene present in the host and that which was introduced. Amplification and sequencing with primers wbeTfor 87> (SEQ ID NO 14: 5'-CGGTGCAAACGTTGGAACTTTCTG-3') and wbeT rev 51< (SEQ ID NO 15: 5'-GGAAAACAATGCCATCCAAATTCGC-3') that only allow amplification if there are tandem copies of the *wbeT* gene successfully amplified the 3' end of the proximal gene (from amino acid 87) and the 5' end of the distal gene up to amino acid 51 and the plasmid in between. Sequencing using the wbeTfor 87> primer showed that in the strain 1342 the *wbeT* gene adjacent to the native promoter was the truncated host gene. The distal gene had the wild-type sequence but no promoter. This arrangement led to the Hikojima phenotype since the wild-type gene was being expressed at extremely low levels from a cryptic promoter.

In the Ogawa strain 1356 the arrangement was different. The recombination had resulted in the native *wbeT* gene being expressed from the native promoter and the mutant S158G gene being placed distally and therefore having no promoter recognizable at all. Both copies of the gene appear to have lost the stop codon at position 219, but this mutation had no apparent influence on the phenotype.

### Example 4: Genetically modified Vibrio cholerae cells expressing O1 antigens of both Ogawa and Inaba serotypes by expressing low levels of native WbeT-protein

In conjunction with the experiments on mutant *wbeT* described in Example 2 it was noted that a control plasmid carrying the wild-type *wbeT* was able to partially complement the mutant gene in strain JS1569 even when it was not induced. This resulted in a Hikojima serotype even in the presence of the wild type gene demonstrating that the phenotype can also be achieved by limiting the levels of expression; in this case by keeping the *tac* promoter repressed and allowing only breakthrough expression that occurs in the absence of inducer.

In Example 3, the clone 1342 had a chromosomally integrated wild-type gene expressed from a cryptic promoter, which resulted in a Hikojima serotype. These results confirm that the expression of the wild-type gene at very low levels can result in the Hikojima serotype.

### Example 5: Characterization of the phenotype of genetically modified Vibrio cholerae cells

*V. cholerae* bacteria of strain JS1569 (Inaba) that had been modified to contain plasmids encoding for either the wild-type *WbeT* methylase protein (strain JS1569/S158S) or a mutated *wbeT* gene encoding for *WbeT* protein with a mutation in position 158 from S to G (JS1569/S158G) or from S to A (JS1569/S158A) were grown on LB agar plates, and single colonies were tested for agglutination by antibodies specific for Inaba and Ogawa O antigens, respectively.

These antibodies were obtained after first immunizing rabbits with purified Ogawa and Inaba LPS, respectively and then extensively absorbing the sera with formalin killed bacteria of the heterologous serotype in order to remove cross-reactive antibodies. After absorption, Ogawa-specific antiserum gave strong agglutination of reference *V. cholerae* cells of the Ogawa serotype but could not agglutinate Inaba cells and *vice versa* for the Inaba specific serum.

Agglutination tests were performed by standard method. Briefly, a single colony from a fresh plate of the tested strain was suspended in 50-100 µl physiological saline buffer, and 10 µl of the suspension was placed on a microscope slide. Then 10 µl of appropriately diluted specific antiserum was added and mixed with the cells by tilting the slide backwards and forwards for up to 5 minutes until agglutination was clearly visible. Each assay was compared with negative and positive controls consisting of cells from reference Inaba and Ogawa strains.

Additionally, a control for spontaneous agglutination in which serum was replaced by buffer was performed for each strain tested. Results are shown in the table below and show that the JS1569/S158S strain containing plasmid encoding for wild-type *WbeT* protein had completely switched in serotype from Inaba to Ogawa, the JS1569/S158G strain with a *WbeT* 158S to G mutation expressed strong Ogawa but also detectable Inaba reactivity, and JS1569/S158A with a *WbeT* 158S to A mutation had only marginal Ogawa reactivity.

| Strain | *WbeT* encoding Plasmid | Agglutination with anti-Ogawa antibody | Agglutination with anti-Inaba antibody |
|---|---|---|---|
| JS1569 Inaba | None | **-** | **+++** |
| Cairo 50 Ogawa | None | **+++** | **-** |
| A457 Ogawa | None | **+++** | **-** |
| JS1569/S158S | WbeT S158S wild-type | **+++** | **(+)** |
| JS1569/S158G | WbeT S158G | **++** | **++** |
| JS1569/S158A | WbeT S158A | **(+)** | **+++** |

These results were confirmed when formalin-killed bacteria of the same strains were tested for agglutination with the same sera. They were also confirmed and extended when the formalin-killed bacteria were tested for their quantitative expression of Inaba and Ogawa antigens on the bacterial surface using an ELISA-inhibition method. The method was performed as follows: High binding Greiner Bio-one plates were coated with Ogawa LPS by incubating overnight with 100 µl per well of a solution of 5 µg/ml purified Ogawa LPS in PBS. Starting with 200 microliter of OD₆₀₀ 1.00 formalin killed bacteria; seven serial five-fold dilutions were made (down to 1:15625) in PBS with an eighth blank tube containing no cells. 150 microliter of each dilution was mixed together with an equal amount of appropriately diluted anti-Ogawa serum in PBS, 0.2% BSA. The samples were incubated at room temperature for 1 hour without shaking. The coated plates were washed twice with PBS and blocked with 200 µl/well of PBS, 0.1% BSA for 30 minutes at 37° C. The cells were removed from the suspensions by centrifugation for 5 minutes at 20,000 x g and 100 microliter of the supernatants were added to the blocked the plate(s). A blank containing PBS, 0.1% BSA with neither cells nor anti-Ogawa serum was included in all plates and all samples were run in duplicate. Plates were incubated for 1 hour at room temperature and then washed three times with PBS, 0.05% Tween 20. 100 microliter of appropriately diluted goat anti-rabbit IgG in PBS, 0.1% BSA, 0.05% Tween 20 was added to each well and incubated for 1 hour at room temperature. The plate was washed three times with PBS, 0.05% Tween 20 before adding the substrate solution of 0.1% ortpphenylenediamine (OPD) and 0.012% H₂O₂ in 0.1M citrate pH4.5. The absorbance was read at 490nm after 10min.

The bacterial dilution resulting in 50% inhibition was extrapolated and also the percentage inhibition of the absorbance at bacterial dilution of 1:25 with the results shown in the table below. The results show that the JS1569/S158S strain containing a *wbeT* gene encoding for wild-type *WbeT could* efficiently inhibit the specific anti-Ogawa serum, whereas the strains containing *wbeT* genes with single mutations could also inhibit the anti-Ogawa serum with intermediate activity (JS1569/S158G) or just detectably (JS1569/S158A):

| Strain | Dilution for 50% inhibition | % inhibition at dilution 1:25 |
|---|---|---|
| JS1569 Inaba | **<< 1:1** | **0** |
| A457 Ogawa | **1:60** | **65** |
| JS1569/S158G | **1:70** | **70** |
| JS1569/S158S | **1:1** | **20** |

Based on the plasmids encoding for *WbeT* and S158G, respectively, two recombinant derivative strains of JS1569, 1356 (wild-type *WbeT)* and 1342 (S158G *WbeT*), were obtained where the mutated *wbeT* gene had been stably inserted into the chromosome, giving unexpected but stable phenotypes. For an explanation and description of these strains see examples above. These strains and appropriate reference strains were subjected to colony blots for assaying Ogawa expression. Strains containing mutated *wbeT* genes were patched out on an LB agar plate together with Inaba and Ogawa control strains and grown overnight at 37° C. A nitrocellulose membrane moistened with PBS was applied on the plate with the grown out colonies and was left for 15 minutes at room temperature. The membrane was left to dry on a piece of paper for 5 minutes before it was blocked twice for 20 minutes in 10 ml of 1% Bovine serum albumin (BSA) in PBS at room temperature. The blocking liquid was discarded and replaced with an appropriate dilution of the anti-Ogawa serum in 10 ml in PBS containing 0.1% BSA and 0.05% Tween 20. The membrane was incubated at room temperature on a rocking table for 2 hours. The membrane was then washed three times with PBS containing 0.05% Tween20 before adding goat anti-rabbit IgG conjugated to horseradish peroxidase (Jackson Immunoresearch Laboratories Inc.) in 0.1% BSA in PBS with 0.05%Tween20 and incubating for 2 hours at room temperature with gentle agitation.

Following a further three washes in PSB, 0.05%Tween 20 and a single wash with PBS alone the membrane was developed for 15 minutes with 0.05% 4-chloro-1-naphtol and 0.015% H₂O₂ in 20 mM Tris -HCl pH 7.5 containing 500 mM NaCl and 16.7% methanol. It was then washed thoroughly with tap water and was left to dry on a piece of paper. A digital photo was taken of the developed membrane and the staining density measured with a computer system.

Results in the table show that strain 1356 expressed almost as much Ogawa antigen as the Ogawa reference strain whereas strain 1342 expressed substantially lesser amounts of Ogawa antigen. These findings were further confirmed when formalin-killed preparations of the strains were tested for quantitative expression of Ogawa antigen by inhibition-ELISA done as described above as also shown in the table:

| **Strain** | **Dot blot density units/mm²** | **Dilution for 50% inhibition** |
|---|---|---|
| A 457 Ogawa | **12700** | **1:60** |
| JS1569 Inaba | **0** | **<<1:1** |
| 1356 | **10000** | **1:80** |
| 1342 | **4500** | **1:7** |

### Example 6: Genetic modification of the endogenous wbeT gene to obtain Hikojima serotype

Although the strains presented in the above examples are stable and clearly have the desired phenotypes, an alternative manner of obtaining a Hikojima phenotype is to perform true gene substitutions in the endogenous gene. Suitable mutations (as disclosed above) at position 158 of the endogenous, active *wbeT* gene product also result in diminished activity and therefore a Hikojima serotype. This will result in a range of mutants with differing levels of Ogawa expression that can be tested for Hikojima expression as in Example 5 and in immunization experiments for the optimal immunogenic properties.

As mentioned, the pMT-SUICIDE vector lacks a suitable counter-selection gene and it has proved difficult isolate derivatives that have lost the plasmid and retain the desired gene and phenotype. For this reason, a new suicide vector was constructed carrying the *sacB* gene from *Bacillus subtilis,* allowing strains that have lost the plasmid to be isolated by selection on plates containing sucrose since expression of the *sacB* gene product in gram-negative bacteria is lethal and only colonies derived from cells that have lost the plasmid due to homologous recombination between the two copies of the *wbeT* gene will survive. The plasmid, pMT Suicide/sacB (SEQ ID NO: 16) is much smaller than comparable plasmids with the same functions and is much easier to use.

The wild-type and a number of mutant *wbeT* genes have been cloned into the new vector and will be mated into the strain JS1569 as before, as well as to other strains such as a suitable Ogawa strain.

In order to optimize the chances of obtaining a correct clone the gene arrangement of the partial diploids that resulting from the transconjugation experiments will be analysed before a candidate strain is chosen for taking further for selection of strains that have recombined out the plasmid.

### Example 7: Expression of hybrid CFA/I+CS2

It has recently been shown that *E. coli* TOP10 and *V. cholerae* JS1569 strains can express one of the major colonization factor of ETEC, i.e. CFA/I fimbriae, on their surface (Tobias J, Lebens M, Bölin I, Wiklund G, Svennerholm AM. Vaccine. 2008 Feb 6;26(6):743-52). Following electroporation of a CFA/I-containing expression vector into the above strains, the surface expression was detected by dot blot assay.

It has also recently been shown that *E. coli* TOP10 is capable of expressing a hybrid fimbriae containing the major subunits of both CFA/I and CS2- i.e. an additional major colonization factor of ETEC (Tobias J, Svennerholm AM, Holmgren J, Lebens M. Appl Microbiol Biotechnol. 2010 Jul;87(4))

Therefore, we examined the feasibility of expression of the same hybrid fimbriae on *V. cholerae* JS1569. The strain was electroporated with the expression vector pJT-CFA/I-CotA as described (Tobias et al. 2008, *supra;* Tobias J, Holmgren J, Hellman M, Nygren E, Lebens M, Svennerholm AM. Vaccine. 2010 Aug 20. [Epub ahead of print]) Fifty colonies (clones) were then streaked on two LB plates supplemented with chloramphenicol (12.5 µg/ml) and IPTG (1 mM), followed by over-night incubation at 37°C. Specific monoclonal antibodies (MAb) 1:6 against CFA/I and MAb 10:3 against CS2 were then applied in colony blot assay (Tobias *et al,* 2010, *supra*) to examine the surface expression of the hybrid CFA/I-CotA fimbrie on *V. cholerae* JS1569. The blots were then developed and showed positive signal of surface expression of the hybrid CFA/I-CS2 fimbriae on all tested fifty clones of *V. cholerae* JS1569.

Thus it is possible to combine the expression of antigenic hybrid fimbriae in the same cells that have been engineered to express O1 antigens of both Ogawa and Inaba serotypes.

## Claims

1. A vaccine comprising a *Vibrio cholerae* O1 cell, **characterized in that** said cell comprises O1 antigens of both Ogawa and Inaba serotypes, wherein the vaccine comprises multiple *Vibrio cholerae* O1 cells that comprise O1 antigens of both Ogawa and Inaba serotypes, and wherein on average, 10-90 % of the O1 antigens of the cells are of the Ogawa-serotype.

2. A vaccine according to any of the preceding claims, with the proviso that said vaccine does not contain any further immunogically active whole cells in addition to *Vibrio cholerae* O1 cells that comprise O1 antigens of both Ogawa and Inaba serotypes.

3. A vaccine according to any of the preceding claims, wherein the vaccine is for oral administration.

4. A vaccine according to any of the preceding claims, wherein the cell is formalin-inactivated.

5. A vaccine according to any of the preceding claims, wherein said cell is as defined in any of claims 10-11.

6. A vaccine according to any of the preceding claims, for use in preventive immunization.

7. A vaccine according to any of the preceding claims, for use in preventive immunization against cholera.

8. A vaccine according to any of the preceding claims, wherein the cell further comprises one or more ETEC colonization factor (CF) protein(s), such as CFA/I, CS2 or CS5, wherein said CF protein(s) is/are expressed either as single, double or hybrid fimbriae.

9. A vaccine according to claim 8, for use in preventive immunization against enterotoxigenic *Escherichia coli*-infection (ETEC).

10. A *Vibrio cholerae* O1 cell simultaneously expressing both Inaba and Ogawa antigens **characterized in that**
a. the cell comprises an endogenous *wbeT*-gene; and
b. the cell comprises a recombinant DNA-construct capable of modulating the expression level of endogenous *wbeT* gene or the enzymatic activity of the product thereof;
wherein said construct is adapted to modifying the endogenous *wbeT* gene of the host by means of homologous recombination and wherein
c. the modulated level of *WbeT* enzyme activity is such that the cell simultaneously expresses Inaba and Ogawa antigens, wherein 10-90 % of the O1 antigen expressed by the cell is of the Ogawa-serotype.

11. A *Vibrio cholerae* O1 cell according to claim 10, wherein the cell further expresses one or more ETEC colonization factor (CF) protein(s), such as CFA/I, CS2 or CS5, wherein said CF protein(s) is/are expressed either as single, double or hybrid fimbriae.

12. A method for manufacturing a vaccine, comprising the steps of:
a. providing a *Vibrio cholerae* O1 cell comprising O1 antigens of both Ogawa and Inaba serotypes; and
b. killing said cell.

13. A method according to claim 12, wherein the killing is performed by formalin-treatment.

14. A method according to any of claims 12-13 wherein the cell is a cell according to any of claims 10-11.

## Patentansprüche

1. Impfstoff, umfassend eine *Vibrio cholerae* 01-Zelle, **dadurch gekennzeichnet, dass** die Zelle O1 Antigene der Serotypen Ogawa und Inaba umfasst, wobei der Impfstoff mehrere *Vibrio cholerae* 01-Zellen umfasst, die Antigene der Serotypen Ogawa und Inaba umfassen, und wobei durchschnittlich 10 bis 90 % der O1-Antigene der Zellen vom Serotyp Ogawa sind.

2. Impfstoff nach einem der vorhergehenden Ansprüche, mit der Vorgabe, dass der Impfstoff zusätzlich zu *Vibrio cholerae* O1 Zellen, die Antigene der Serotypen Ogawa und Inaba umfassen, keine weiteren immunologisch aktiven ganzen Zellen enthält.

3. Impfstoff nach einem der vorhergehenden Ansprüche, wobei der Impfstoff für eine orale Verabreichung bestimmt ist.

4. Impfstoff nach einem der vorhergehenden Ansprüche, wobei die Zelle mit Formalin inaktiviert ist.

5. Impfstoff nach einem der vorhergehenden Ansprüche, wobei die Zelle der Definition nach einem der Ansprüche 10 bis 11 entspricht.

6. Impfstoff nach einem der vorhergehenden Ansprüche zur Verwendung bei einer Schutzimpfung.

7. Impfstoff nach einem der vorhergehenden Ansprüche zur Verwendung bei einer Schutzimpfung gegen Cholera.

8. Impfstoff nach einem der vorhergehenden Ansprüche, wobei die Zelle ferner mindestens ein ETEC-Besiedelungsfaktor(CF)-Protein umfasst, wie beispielsweise CFA/I, CS2 oder CS5, wobei das CF-Protein bzw. die CF-Proteine als Einzel-, Doppel- oder Hybridfimbrien exprimiert ist bzw. sind.

9. Impfstoff nach Anspruch 8 zur Verwendung bei einer Schutzimpfung gegen eine Infektion mit enterotoxigenen Escherichia coli (ETEC).

10. *Vibrio cholerae* 01-Zelle, die simultan sowohl Inaba- als auch Ogawa-Antigene exprimiert, **dadurch gekennzeichnet, dass**
a. die Zelle ein endogenes wbeT-Gen aufweist; und
b. die Zelle ein rekombinantes DNA-Konstrukt aufweist, das zum Modulieren des Expressionsgrades eines endogenen wbeT-Gens oder der enzymatischen Aktivität von dessen Produkt in der Lage ist;
wobei das Konstrukt angepasst ist, um das endogene wbeT-Gen des Wirts durch homologe Rekombination zu modifizieren;
und wobei
c. das modulierte Niveau der Aktivität des WbeT-Enzyms derart ist, dass die Zelle simultan Inaba- und Ogawa-Antigene exprimiert, wobei 10 bis 90 % des von der Zelle exprimierten 01-Antigens vom Serotyp Ogawa sind.

11. *Vibrio cholerae* 01-Zelle nach Anspruch 10, wobei die Zelle ferner mindestens ein ETEC-Besiedelungsfaktor(CF)-Protein exprimiert, wie beispielsweise CFA/I, CS2 oder CS5, wobei das CF-Protein bzw. die CF-Proteine als Einzel-, Doppel- oder Hybridfimbrien exprimiert ist bzw. sind.

12. Verfahren zum Herstellen eines Impfstoffs, umfassend die Schritte:
a. Bereitstellen einer *Vibrio cholerae* 01-Zelle, umfassend 01-Antigene der Serotypen Ogawa und Inaba; und
b. Töten der Zelle.

13. Verfahren nach Anspruch 12, wobei das Töten durch eine Behandlung mit Formalin durchgeführt wird.

14. Verfahren nach einem der Ansprüche 12 bis 13, wobei es sich bei der Zelle um eine Zelle nach einem der Ansprüche 10 bis 11 handelt.

## Revendications

1. Vaccin comprenant une cellule de *Vibrio cholerae* O1, **caractérisé en ce que** ladite cellule comprend des antigènes O1 des sérotypes Ogawa et Inaba, le vaccin comprenant de multiples cellules de *Vibrio cholerae* O1 qui comprennent des antigènes O1 des sérotypes Ogawa et Inaba, et dans lequel, en moyenne, 10 à 90 % des antigènes O1 des cellules sont du sérotype Ogawa.

2. Vaccin selon l'une quelconque des revendications précédentes, avec pour restriction que ledit vaccin ne contient pas d'autres cellules immunologiquement actives que les cellules de *Vibrio cholerae* O1 comprenant des antigènes O1 des sérotypes Ogawa et Inaba.

3. Vaccin selon l'une quelconque des revendications précédentes, dans lequel le vaccin est destiné à une administration orale.

4. Vaccin selon l'une quelconque des revendications précédentes, dans lequel la cellule est inactivée par la formaline.

5. Vaccin selon l'une quelconque des revendications précédentes, dans lequel la cellule est une cellule selon l'une quelconque des revendications 10 et 11.

6. Vaccin selon l'une quelconque des revendications précédentes, destiné à une immunisation préventive.

7. Vaccin selon l'une quelconque des revendications précédentes, destiné à une immunisation préventive contre le choléra.

8. Vaccin selon l'une quelconque des revendications précédentes, dans lequel la cellule comprend en outre une ou plusieurs protéines facteurs de colonisation (CF) d'ETEC, telles que CFA/I, CS2 ou CS5, lesdites protéines CF étant exprimées sous la forme de fimbriae simples, doubles ou bien hybrides.

9. Vaccin selon la revendication 8, destiné à une immunisation préventive contre une infection par *Escherichia coli* entérotoxinogène (ECET).

10. Cellule de *Vibrio cholerae* O1 exprimant simultanément les antigènes Inaba et Ogawa, **caractérisée en ce que**
a. la cellule comprend un gène *wbeT* endogène ; et
b. la cellule comprend une construction d'ADN recombinant apte à moduler le niveau d'expression du gène *wbeT* endogène ou l'activité enzymatique de son produit ;
ladite construction étant conçue pour modifier le gène *wbeT* endogène de l'hôte au moyen d'une recombinaison homologue et dans laquelle
c. le niveau modulé de l'activité enzymatique de *WbeT est* tel que la cellule exprime simultanément les antigènes Inaba et Ogawa, 10 à 90 % de l'antigène O1 exprimé par la cellule étant du sérotype Ogawa.

11. Cellule de *Vibrio cholerae* O1 selon la revendication 10, dans lequel la cellule exprime en outre une ou plusieurs protéines facteurs de colonisation (CF) d'ETEC, telles que CFA/I, CS2 ou CS5, lesdites protéines CF étant exprimées sous la forme de fimbriae simples, doubles ou bien hybrides.

12. Procédé de production d'un vaccin, comprenant les étapes consistant à :
a. disposer d'une cellule de *Vibrio cholerae* O1 comprenant des antigènes O1 des sérotypes Ogawa et Inaba ; et
b. inactiver ladite cellule.

13. Procédé selon la revendication 12, dans lequel l'inactivation s'effectue par traitement à la formaline.

14. Procédé selon l'une quelconque des revendications 12 et 13, dans lequel la cellule est une cellule selon l'une quelconque des revendications 10 et 11.


### SEQUENCE LISTING

<110> Gotovax AB Holmgren, Jan Lebens, Michael
<120> Vaccine against cholera and enterotoxigenic E. coli (ETEC) diarrhea
<130> P40903745PCT00
<150> US 61/272351
   <151> 2009-09-16
<160> 16
<170> PatentIn version 3.5
<210> 1
   <211> 41
   <212> DNA
   <213> Vibrio cholerae
<400> 1
   cccggtctcg aattcctgca tctgcaagtt gattctgtat g 41
<210> 2
   <211> 40
   <212> DNA
   <213> Vibrio cholerae
<400> 2
   cccggtctca agcttatagt gaactcttcg gaaatgtctg 40
<210> 3
   <211> 26
   <212> DNA
   <213> Vibrio cholerae
<400> 3
   ctgcatctgc aagttgattc tgtatg 26
<210> 4
   <211> 25
   <212> DNA
   <213> Vibrio cholerae
<400> 4
   atagtgaact cttcggaaat gtctg 25
<210> 5
   <211> 953
   <212> DNA
   <213> Vibrio cholerae
<220>
   <221> CDS
   <222> (67)..(927)
<400> 5
   cctgcatctg caagttgatt ctgtatgtta ttttttacgc taatattatt taaaattgag       60
   gtagta atg aaa cat cta ata aaa aac tat gta caa aaa tta att aaa        108
          Met Lys His Leu Ile Lys Asn Tyr Val Gln Lys Leu Ile Lys
          1               5                   10
   aca gag ctt gat gct att cag tca aag tct gtt cat gat aat cga aac       156
   Thr Glu Leu Asp Ala Ile Gln Ser Lys Ser Val His Asp Asn Arg Asn
   15                  20                  25                  30
   ttc att tac aat gga gag ttt tta att ctt gaa agc gaa ttt gga tgg       204
   Phe Ile Tyr Asn Gly Glu Phe Leu Ile Leu Glu Ser Glu Phe Gly Trp
                   35                  40                  45
   cat tgt ttt ccc aga gtg cag ttg aac cat gct tta agc tac aaa aac       252
   His Cys Phe Pro Arg Val Gln Leu Asn His Ala Leu Ser Tyr Lys Asn
               50                  55                  60
   cca aac ttt gat tta ggt atg cgt cac tgg att gtt aat cat tgt aag       300
   Pro Asn Phe Asp Leu Gly Met Arg His Trp Ile Val Asn His Cys Lys
           65                  70                  75
   cat gac acc act tat att gat atc ggt gca aac gtt gga act ttc tgt       348
   His Asp Thr Thr Tyr Ile Asp Ile Gly Ala Asn Val Gly Thr Phe Cys
       80                  85                  90
   gga atc gct gct cgt cat att aca caa gga aaa att ata gcg ata gaa       396
   Gly Ile Ala Ala Arg His Ile Thr Gln Gly Lys Ile Ile Ala Ile Glu
   95                  100                 105                 110
   cca ctc aca gaa atg gaa aat agt att agg atg aat gtt caa tta aat       444
   Pro Leu Thr Glu Met Glu Asn Ser Ile Arg Met Asn Val Gln Leu Asn
                   115                 120                 125
   aat cca ctg gtt gag ttt cat cat ttt ggc tgt gca ata ggt gag aat       492
   Asn Pro Leu Val Glu Phe His His Phe Gly Cys Ala Ile Gly Glu Asn
               130                 135                 140
   gaa ggg gaa aat att ttc gaa gtt tat gag ttt gat aat agg gtg tca       540
   Glu Gly Glu Asn Ile Phe Glu Val Tyr Glu Phe Asp Asn Arg Val Ser
           145                 150                 155
   tca tta tat ttt caa aaa aat aca gac ata gca gat aag gtt aaa aat       588
   Ser Leu Tyr Phe Gln Lys Asn Thr Asp Ile Ala Asp Lys Val Lys Asn
       160                 165                 170
   agc caa gtt ctg gtt aga aag tta agt agt tta gat ata tcg cct act       636
   Ser Gln Val Leu Val Arg Lys Leu Ser Ser Leu Asp Ile Ser Pro Thr
   175                 180                 185                 190
   aac tct gta gtt ata aaa att gat gct gaa ggc gca gaa ata gag ata       684
   Asn Ser Val Val Ile Lys Ile Asp Ala Glu Gly Ala Glu Ile Glu Ile
                   195                 200                 205
   tta aac cag att tac gaa ttc aca gaa aag cat aat gga att gaa tat       732
   Leu Asn Gln Ile Tyr Glu Phe Thr Glu Lys His Asn Gly Ile Glu Tyr
               210                 215                 220
   tat att tgc ttt gaa ttt gca atg ggt cat ata cag agg tct aat aga       780
   Tyr Ile Cys Phe Glu Phe Ala Met Gly His Ile Gln Arg Ser Asn Arg
           225                 230                 235
   act ttt gat gag att ttt aac ata ata aac tca aaa ttc gga agt aag       828
   Thr Phe Asp Glu Ile Phe Asn Ile Ile Asn Ser Lys Phe Gly Ser Lys
       240                 245                 250
   gca tat ttt att cat cca tta tca tcc gct gaa cat cct gag ttt aat         876
   Ala Tyr Phe Ile His Pro Leu Ser Ser Ala Glu His Pro Glu Phe Asn
   255                 260                 265                 270
   aaa gca acg cag gat att aat ggg aat atc tgt ttt aaa tat gta tca         924
   Lys Ala Thr Gln Asp Ile Asn Gly Asn Ile Cys Phe Lys Tyr Val Ser
                   275                 280                 285
   taa aataatttaa tatattccgt atgtca                                        953
<210> 6
   <211> 286
   <212> PRT
   <213> Vibrio cholerae
<400> 6
   Met Lys His Leu Ile Lys Asn Tyr Val Gln Lys Leu Ile Lys Thr Glu
   1               5                   10                  15
   Leu Asp Ala Ile Gln Ser Lys Ser Val His Asp Asn Arg Asn Phe Ile
               20                  25                  30
   Tyr Asn Gly Glu Phe Leu Ile Leu Glu Ser Glu Phe Gly Trp His Cys
           35                  40                  45
   Phe Pro Arg Val Gln Leu Asn His Ala Leu Ser Tyr Lys Asn Pro Asn
       50                  55                  60
   Phe Asp Leu Gly Met Arg His Trp Ile Val Asn His Cys Lys His Asp
   65                  70                  75                  80
   Thr Thr Tyr Ile Asp Ile Gly Ala Asn Val Gly Thr Phe Cys Gly Ile
                   85                  90                  95
   Ala Ala Arg His Ile Thr Gln Gly Lys Ile Ile Ala Ile Glu Pro Leu
               100                 105                 110
   Thr Glu Met Glu Asn Ser Ile Arg Met Asn Val Gln Leu Asn Asn Pro
           115                 120                 125
   Leu Val Glu Phe His His Phe Gly Cys Ala Ile Gly Glu Asn Glu Gly
       130                 135                 140
   Glu Asn Ile Phe Glu Val Tyr Glu Phe Asp Asn Arg Val Ser Ser Leu
   145                 150                 155                 160
   Tyr Phe Gln Lys Asn Thr Asp Ile Ala Asp Lys Val Lys Asn Ser Gln
                   165                 170                 175
   Val Leu Val Arg Lys Leu Ser Ser Leu Asp Ile Ser Pro Thr Asn Ser
               180                 185                 190
   Val Val Ile Lys Ile Asp Ala Glu Gly Ala Glu Ile Glu Ile Leu Asn
           195                 200                 205
   Gln Ile Tyr Glu Phe Thr Glu Lys His Asn Gly Ile Glu Tyr Tyr Ile
       210                 215                 220
   Cys Phe Glu Phe Ala Met Gly His Ile Gln Arg Ser Asn Arg Thr Phe
   225                 230                 235                 240
   Asp Glu Ile Phe Asn Ile Ile Asn Ser Lys Phe Gly Ser Lys Ala Tyr
                   245                 250                 255
   Phe Ile His Pro Leu Ser Ser Ala Glu His Pro Glu Phe Asn Lys Ala
               260                 265                 270
   Thr Gln Asp Ile Asn Gly Asn Ile Cys Phe Lys Tyr Val Ser
           275                 280                 285
<210> 7
   <211> 4858
   <212> DNA
   <213> Artificial Sequence
<220>
   <223> Synthetic plasmid
<400> 7
   aggtggcact tttcggggaa atgtgcgcgg aacccctatt tgtttatttt tctaaataca      60
   ttcaaatatg tatccgctca tgagacaata accctgataa atgcttcaat aatccggatt     120
   gaaaaaggaa gagtatgagt attcaacatt tccgtgtcgc ccttattccc ttttttgcgg     180
   cattttgcct tcctgttttt gctcacccag aaacgctggt gaaagtaaaa gatgctgaag     240
   atcagttggg tgcacgagtg ggttacatcg aactggatct caacagcggt aagatccttg     300
   agagttttcg ccccgaagaa cgttttccaa tgatgagcac ttttaaagtt ctgctatgtg     360
   gcgcggtatt atcccgtgtt gacgccgggc aagagcaact cggtcgccgc atacactatt     420
   ctcagaatga cttggttgag tactcaccag tctcagaaaa gcatcttacg gatggcatga     480
   cagtaagaga attatgcagt gctgccataa ccatgagtga taacactgcg gccaacttac     540
   ttctgacaac gatcggagga ccgaaggagc taaccgcttt tttgcacaac atgggggatc     600
   atgtaactcg ccttgatcgt tgggaaccgg agctgaatga agccatacca aacgacgagc     660
   gtgacaccac gatgcctgta gcaatggcaa caacgttgcg caaactatta actggcgaac     720
   tacttactct agcttcccgg caacaattaa tagactggat ggaggcggat aaagttgcag     780
   gaccacttct gcgctcggcc cttccggctg gctggtttat tgctgataaa tctggagccg     840
   gtgagcgtgg gtctcgcggt atcattgcag cactggggcc agatggtaag ccctcccgta     900
   tcgtagttat ctacacgacg gggagtcagg caactatgga tgaacgaaat agacagatcg      960
   ctgagatagg tgcctcactg attaagcatt ggtaactgtc agaccaagtt tactcatata     1020
   tactttagat tgatttaaaa cttcattttt aatttaaaag gatctaggtg aagatccttt     1080
   ttgataatct catgaccaaa atcccttaac gtgagttttc gttccactca gcgtcagacc     1140
   ccgtagaaaa gatcaaagga tcttcttgag atcctttttt tctgcgcgta atctgctgct     1200
   tgcaaacaaa aaaaccaccg ctaccagcgg tggtttgttt gccggatcaa gagctaccaa     1260
   ctctttttcc gaaggtaact ggcttcagca gagcgcagat accaaatact gtccttctag     1320
   tgtagccgta gttaggccac cacttcaaga actctgtagc accgcctaca tacctcgctc     1380
   tgctaatcct gttaccagtg gctgctgcca gtggcgataa gtcgtgtctt accgggttgg     1440
   actcaagacg atagttaccg gataaggcgc agcggtcggg ctgaacgggg ggttcgtgca     1500
   cacagcccag cttggagcga acgacctaca ccgaactgag atacctacag cgtgagcatt     1560
   gagaaagcgc cacgcttccc gaagggagaa aggcggacag gtatccggta agcggcaggg     1620
   tcggaacagg acagcgcacg agggagcttc cagggggaaa cgcctggtat ctttatagtc     1680
   ctgtcgggtt tcgccacctc tgacttgagc gtcgattttt gtgatgctcg tcaggggggc     1740
   ggagcctatg gaaaatcttt cctgcgttat cccctgattc tgtggataac cgtattaccg     1800
   cctttgagtg agctgacgcc agcaacgcgg cctttttacg gttcctggcc ttttgctggc     1860
   cttttgctca catgttcttt cctgcgttat cccctgattc tgtggataac cgtattaccg     1920
   cctttgagtg agctgatacc gctcgccgca gccgaacgac cgagcgcagc gagtcagtga     1980
   gcgaggaagc ggaagagcgc ctgatgcggt attttctcct tacgcatctg tgcggtattt     2040
   cacaccgcat atggtgcact ctcagtacaa tctgctctga tgccgcatag ttaagccagt     2100
   atacactccg ctatcgctac gtgactgggt catggctgcg ccccgacacc cgccaacacc     2160
   cgctgacgcg ccctgacggg cttgtctgct cccggcatcc gcttacagac aagctgtgac     2220
   cgtctccggg agctgcatgt gtcagaggtt ttcaccgtca tcaccgaaac gcgcgaggca     2280
   ggatcccgaa cgccagcaag acgtagccca gcgcgtcggc cagcttgcaa ttcgcgctaa     2340
   ctcacattaa ttgcgttgcg ctcactgccc gctttccagt cgggaaacct gtcgtgccag     2400
   ctgcattaat gaatcggcca acgcgcgggg agaggcggtt tgcgtattgg gcgccagggt     2460
   ggtttttctt ttcaccagtg agacgggcaa cagctgattg cccttcaccg cctggccctg     2520
   agagagttgc agcaagcggt ccacgctggt ttgccccagc aggcgaaaat cctgtttgat     2580
   ggtggttaac ggcgggatat aacatgagct gtcttcggta tcgtcgtatc ccactaccga     2640
   gatatccgca ccaacgcgca gcccggactc ggtaatggcg cgcattgcgc ccagcgccat     2700
   ctgatcgttg gcaaccagca tcgcagtggg aacgatgccc tcattcagca tttgcatggt     2760
   ttgttgaaaa ccggacatgg cactccagtc gccttcccgt tccgctatcg gctgaatttg     2820
   attgcgagtg agatatttat gccagccagc cagacgcaga cgcgccgaga cagaacttaa     2880
   tgggcccgct aacagcgcga tttgctggtg acccaatgcg accagatgct ccacgcccag 2940
   tcgcgtaccg tcttcatggg agaaaataat actgttgatg ggtgtctggt cagagacatc 3000
   aagaaataac gccggaacat tagtgcaggc agcttccaca gcaatggcat cctggtcatc 3060
   cagcggatag ttaatgatca gcccactgac gcgttgcgcg agaagattgt gcaccgccgc 3120
   tttacaggct tcgacgccgc ttcgttctac catcgacacc accacgctgg cacccagttg 3180
   atcggcgcga gatttaatcg ccgcgacaat ttgcgacggc gcgtgcaggg ccagactgga 3240
   ggtggcaacg ccaatcagca acgactgttt gcccgccagt tgttgtgcca cgcggttggg 3300
   aatgtaattc agctccgcca tcgccgcttc cactttttcc cgcgttttcg cagaaacgtg 3360
   gctggcctgg ttcaccacgc gggaaacggt ctgataagag acaccggcat actctgcgac 3420
   atcgtataac gttactggtt tcacattcac caccctgaat tgactctctt ccgggcgcta 3480
   tcatgccata ccgcgaaagg ttttgcacca ttcgatggtg tcaacgtaaa tgccgcttcg 3540
   ccttcgcgcg cgaattgcaa gctgatccgg gcttatcgac tgcacggtgc accaatgctt 3600
   ctggcgtcag gcagccatcg gaagctgtgg tatggctgtg caggtcgtaa atcactgcat 3660
   aattcgtgtc gctcaaggcg cactcccgtt ctggataatg ttttttgcgc cgacatcata 3720
   acggttctgg cagatctgaa atgagctgtt gacaattaat catcggctcg tataatgtgt 3780
   ggaattgtga gcggataaca atttcacaca ggaaacagaa ttcctgcatc tgcaagttga 3840
   ttctgtatgt tattttttac gctaatatta tttaaaattg aggtagtatg aaacatctaa 3900
   taaaaaacta tgtacaaaaa ttaattaaaa cagagcttga tgctattcag tcaaagtctg 3960
   ttcatgataa tcgaaacttc atttacaatg gagagttttt aattcttgaa agcgaatttg 4020
   gatggcattg ttttcccaga gtgcagttga accatgcttt aagctacaaa aacccaaact 4080
   ttgatttagg tatgcgtcac tggattgtta atcattgtaa gcatgacacc acttatattg 4140
   atatcggtgc aaacgttgga actttctgtg gaatcgctgc tcgtcatatt acacaaggaa 4200
   aaattatagc gatagaacca ctcacagaaa tggaaaatag tattaggatg aatgttcaat 4260
   taaataatcc actagttgag tttcatcatt ttggctgtgc aataggtgag aatgaagggg 4320
   aaaatatttt cgaagtttat gagtttgata atagggtgtc atcattatat tttcaaaaaa 4380
   atacagacat agcagataag gttaaaaata gccaagttct ggttagaaag ttaagtagtt 4440
   tagatatatc gcctactaac tctgtagtta taaaaattga tgctgaaggc gcagaaatag 4500
   agatattaaa ccagatttac gaattcacag aaaagcataa tggaattgaa tattatattt 4560
   gctttgaatt tgcaatgggt catatacaga ggtctaatag aacttttgat gagattttta 4620
   acataataaa ctcaaaattc ggaagtaagg catattttat tcatccatta tcatccgctg 4680
   aacatcctga gtttaataaa gcaacgcagg atattaatgg gaatatctgt tttaaatatg 4740
   tatcataaaa taatttaata tattctcgta tgtcattgca agttcaacag acatttccga 4800
   agagttcact ataagcttag cccgcctaat gagcgggctt ttttttctcg aggacgtc 4858
<210> 8
   <211> 28
   <212> DNA
   <213> Vibrio cholerae
<400> 8
   gcgcgccaga acttggctat ttttaacc 28
<210> 9
   <211> 104
   <212> DNA
   <213> Vibrio cholerae
<220>
   <221> misc_feature
   <222> (36)..(37)
   <223> n denotes a, t, c or g
<220>
   <221> misc_feature
   <222> (38)..(38)
   <223> b denotes c or g or t
<400> 9
   gggggttcga agtttatgag tttgataata gggtgnnbtc attatatttt caaaaaaata 60
   cagacatagc agataaggtt aaaaatagcc aagttctggc gcgc 104
<210> 10
   <211> 934
   <212> DNA
   <213> Vibrio cholerae
<220>
   <221> CDS
   <222> (22)..(678)
<400> 10
   attatttaaa ttgaggtagt a atg aaa cat cta ata aaa aac tat gta caa          51
                           Met Lys His Leu Ile Lys Asn Tyr Val Gln
                           1               5                   10
   aaa tta att aaa aca gag ctt gat gct att cag tca aag tct gtt cat          99
   Lys Leu Ile Lys Thr Glu Leu Asp Ala Ile Gln Ser Lys Ser Val His
                   15                  20                  25
   gat aat cga aac ttc att tac aat gga gag ttt tta att ctt gaa agc         147
   Asp Asn Arg Asn Phe Ile Tyr Asn Gly Glu Phe Leu Ile Leu Glu Ser
               30                  35                  40
   gaa ttt gga ttg cat tgt ttt ccc aga gtg cag ttg aac cat gct tta         195
   Glu Phe Gly Leu His Cys Phe Pro Arg Val Gln Leu Asn His Ala Leu
           45                  50                  55
   agc tac aaa aac cca aac ttt gat tta ggt atg cgt cac tgg att gtt         243
   Ser Tyr Lys Asn Pro Asn Phe Asp Leu Gly Met Arg His Trp Ile Val
       60                  65                  70
   aat cat tgt aag cat gac acc act tat att gat atc ggt gca aac gtt        291
   Asn His Cys Lys His Asp Thr Thr Tyr Ile Asp Ile Gly Ala Asn Val
   75                  80                  85                  90
   gga act ttc tgt gga atc gct gct cgt cat ata cac caa gga aaa att        339
   Gly Thr Phe Cys Gly Ile Ala Ala Arg His Ile His Gln Gly Lys Ile
                   95                  100                 105
   ata gcg ata gaa cca ctc aca gaa atg gaa aat agt att agg atg aat        387
   Ile Ala Ile Glu Pro Leu Thr Glu Met Glu Asn Ser Ile Arg Met Asn
               110                 115                 120
   gtt caa tta aat aat cca cta gtt gag ttt cat cat ttt ggc tgt gca        435
   Val Gln Leu Asn Asn Pro Leu Val Glu Phe His His Phe Gly Cys Ala
           125                 130                 135
   ata ggt gag aat gaa ggg gaa aat att ttc gaa gtt tat gag ttt gat        483
   Ile Gly Glu Asn Glu Gly Glu Asn Ile Phe Glu Val Tyr Glu Phe Asp
       140                 145                 150
   aat agg gtg tca tca tta tat ttt aaa aaa aat aca gac ata gca gat        531
   Asn Arg Val Ser Ser Leu Tyr Phe Lys Lys Asn Thr Asp Ile Ala Asp
   155                 160                 165                 170
   aag gtt aaa aat agc caa gtt ctg gtt aga aag tta agt agt tta gat        579
   Lys Val Lys Asn Ser Gln Val Leu Val Arg Lys Leu Ser Ser Leu Asp
                   175                 180                 185
   ata tcg cct act aac tct gta gtt ata aaa att gat gct gaa ggc gca        627
   Ile Ser Pro Thr Asn Ser Val Val Ile Lys Ile Asp Ala Glu Gly Ala
               190                 195                 200
   gaa ata gag ata tta aac cag att tac gaa ttc aca gaa aag cat aat        675
   Glu Ile Glu Ile Leu Asn Gln Ile Tyr Glu Phe Thr Glu Lys His Asn
           205                 210                 215
   tga attgaatatt atatttgctt tgaatttgca atgggtcata tacagaggtc             728
   taatagaact tttgatgaga tttttaacat aataaactca aaattcggaa gtaaggcata      788
   ttttattcat ccattatcat ccgctgaaca tcctgagttt aataaagcaa cgcaggatat      848
   taatgggaat atctgtttta aatatgtatc ataaaataat ttaatatatt ccgtatgtca      908
   ttgcaagttc aacagacatt tcgaga                                           934
<210> 11
   <211> 218
   <212> PRT
   <213> Vibrio cholerae
<400> 11
   Met Lys His Leu Ile Lys Asn Tyr Val Gln Lys Leu Ile Lys Thr Glu
   1               5                   10                  15
   Leu Asp Ala Ile Gln Ser Lys Ser Val His Asp Asn Arg Asn Phe Ile
               20                  25                  30
   Tyr Asn Gly Glu Phe Leu Ile Leu Glu Ser Glu Phe Gly Leu His Cys
           35                  40                  45
   Phe Pro Arg Val Gln Leu Asn His Ala Leu Ser Tyr Lys Asn Pro Asn
       50                  55                  60
   Phe Asp Leu Gly Met Arg His Trp Ile Val Asn His Cys Lys His Asp
   65                  70                  75                  80
   Thr Thr Tyr Ile Asp Ile Gly Ala Asn Val Gly Thr Phe Cys Gly Ile
                   85                  90                  95
   Ala Ala Arg His Ile His Gln Gly Lys Ile Ile Ala Ile Glu Pro Leu
               100                 105                 110
   Thr Glu Met Glu Asn Ser Ile Arg Met Asn Val Gln Leu Asn Asn Pro
           115                 120                 125
   Leu Val Glu Phe His His Phe Gly Cys Ala Ile Gly Glu Asn Glu Gly
       130                 135                 140
   Glu Asn Ile Phe Glu Val Tyr Glu Phe Asp Asn Arg Val Ser Ser Leu
   145                 150                 155                 160
   Tyr Phe Lys Lys Asn Thr Asp Ile Ala Asp Lys Val Lys Asn Ser Gln
                   165                 170                 175
   Val Leu Val Arg Lys Leu Ser Ser Leu Asp Ile Ser Pro Thr Asn Ser
               180                 185                 190
   Val Val Ile Lys Ile Asp Ala Glu Gly Ala Glu Ile Glu Ile Leu Asn
           195                 200                 205
   Gln Ile Tyr Glu Phe Thr Glu Lys His Asn
       210                 215
<210> 12
   <211> 1954
   <212> DNA
   <213> Artificial
<220>
   <223> Synthetic plasmid
<400> 12
   agtaatacga ctcactagtg ggcagatctt cgaatgcatc gcgcgcaccg tacgtctcga      60
   ggaattcctg caggatatct ggatccacga agcttcccat ggtgacgtca ccggttctag     120
   atacctaggt gagctctggt accctctagt caaggcctgt cagccgttaa gtgttcctgt     180
   gtcactgaaa attgctttga gaggctctaa gggcttctca gtgcgttaca tccctggctt     240
   gttgtccaca accgttaaac cttaaaagct ttaaaagcct tatatattct tttttttctt    300
   ataaaactta aaaccttaga ggctatttaa gttgctgatt tatattaatt ttattgttca     360
   aacatgagag cttagtacgt gaaacatgag agcttagtac gttagccatg agagcttagt 420
   acgttagcca tgagggttta gttcgttaaa catgagagct tagtacgtta aacatgagag 480
   cttagtacgt gaaacatgag agcttagtac gtactatcaa caggttgaac tgctgatctt 540
   cagatctccg cttgccctca tctgttacgc cggcggtagc cggccagcct cgcagagcag 600
   gattcccgtt gagcaccgcc aggtgcgaat aagggacagt gaagaaggaa cacccgctcg 660
   cgggtgggcc tacttcacct atcctgcccg gctgacgccg ttggatacac caaggaaagt 720
   ctacacgaac cctttggcaa aatcctgtat atcgtgcgaa aaaggatgga tataccgaaa 780
   aaatcgctat aatgaccccg aagcagggtt atgcagcgga aaagcgctgc ttccctgctg 840
   ttttgtggaa tatctaccga ctggaaacag gcaaatgcag gaaattactg aactgagggg 900
   acaggcgaga gatctggcct aggccgaccg aataaatacc tgtgacggaa gatcacttcg 960
   cagaataaat aaatcctggt gtccctgttg ataccgggaa gccctgggcc aacttttggc 1020
   gaaaatgaga cgttgatcgg cacgtaagag gttccaactt tcaccataat gaaataagat 1080
   cactaccggg cgtatttttt gagttgtcga gattttcagg agctaaggaa gctaaaatgg 1140
   agaaaaaaat cactggatat accaccgttg atatatccca atggcatcgt aaagaacatt 1200
   ttgaggcatt tcagtcagtt gctcaatgta cctataacca gaccgttcag ctggatatta 1260
   cggccttttt aaagaccgta aagaaaaata agcacaagtt ttatccggcc tttattcaca 1320
   ttcttgcccg cctgatgaat gctcatccgg aattacgtat ggcaatgaaa gacggtgagc 1380
   tggtgatatg ggatagtgtt cacccttgtt acaccgtttt ccatgagcaa actgaaacgt 1440
   tttcatcgct ctggagtgaa taccacgacg atttccggca gtttctacac atatattcgc 1500
   aagatgtggc gtgttacggt gaaaacctgg cctatttccc taaagggttt attgagaata 1560
   tgtttttcgt ctcagccaat ccctgggtga gtttcaccag ttttgattta aacgtggcca 1620
   atatggacaa cttcttcgcc cccgttttca ccatgggcaa atattatacg caaggcgaca 1680
   aggtgctgat gccgctggcg attcaggttc atcatgccgt ttgtgatggc ttccatgtcg 1740
   gcagaatgct taatgaatta caacagtact gcgatgagtg gcagggcggg gcgtaatttt 1800
   tttaaggcag ttattggtgc ccataaacgc ctggttgcta cgcctgaata agtgataata 1860
   agcggatgaa tggcagaaat tcgaaagcaa attcgacccg gtcgtcggtt cagggcaggg 1920
   tcgttaaata gccgcttatg tctattgctg gttt 1954
<210> 13
   <211> 2948
   <212> DNA
   <213> Artificial
<220>
   <223> Synthetic vector construct with wild-type wbeT insert
<220>
   <221> misc_feature
   <222> (151)..(1011)
   <223> wbeT wild-type CDS insert in reverse orientation
<400> 13
   agtaatacga ctcactagtg ggcagatctt cgaatgcatc gcgcgcaccg tacgtctcga 60
   ggaattcctg caggatatct ggatctatag tgaactcttc ggaaatgtct gttgaacttg 120
   caatgacata cgagaatata ttaaattatt ttatgataca tatttaaaac agatattccc 180
   attaatatcc tgcgttgctt tattaaactc aggatgttca gcggatgata atggatgaat 240
   aaaatatgcc ttacttccga attttgagtt tattatgtta aaaatctcat caaaagttct 300
   attagacctc tgtatatgac ccattgcaaa ttcaaagcaa atataatatt caattccatt 360
   atgcttttct gtgaattcgt aaatctggtt taatatctct atttctgcgc cttcagcatc 420
   aatttttata actacagagt tagtaggcga tatatctaaa ctacttaact ttctaaccag 480
   aacttggcta tttttaacct tatctgctat gtctgtattt ttttgaaaat ataatgatga 540
   caccctatta tcaaactcat aaacttcgaa aatattttcc ccttcattct cacctattgc 600
   acagccaaaa tgatgaaact caactagtgg attatttaat tgaacattca tcctaatact 660
   attttccatt tctgtgagtg gttctatcgc tataattttt ccttgtgtaa tatgacgagc 720
   agcgattcca cagaaagttc caacgtttgc accgatatca atataagtgg tgtcatgctt 780
   acaatgatta acaatccagt gacgcatacc taaatcaaag tttgggtttt tgtagcttaa 840
   agcatggttc aactgcactc tgggaaaaca atgccatcca aattcgcttt caagaattaa 900
   aaactctcca ttgtaaatga agtttcgatt atcatgaaca gactttgact gaatagcatc 960
   aagctctgtt ttaattaatt tttgtacata gttttttatt agatgtttca tactacctca 1020
   attttaaata atattagcgt aaaaaataac atacagaatc aacttgcaga tgcagagatc 1080
   cacgaagctt cccatggtga cgtcaccggt tctagatacc taggtgagct ctggtaccct 1140
   ctagtcaagg cctgtcagcc gttaagtgtt cctgtgtcac tgaaaattgc tttgagaggc 1200
   tctaagggct tctcagtgcg ttacatccct ggcttgttgt ccacaaccgt taaaccttaa 1260
   aagctttaaa agccttatat attctttttt ttcttataaa acttaaaacc ttagaggcta 1320
   tttaagttgc tgatttatat taattttatt gttcaaacat gagagcttag tacgtgaaac 1380
   atgagagctt agtacgttag ccatgagagc ttagtacgtt agccatgagg gtttagttcg 1440
   ttaaacatga gagcttagta cgttaaacat gagagcttag tacgtgaaac atgagagctt 1500
   agtacgtact atcaacaggt tgaactgctg atcttcagat ctccgcttgc cctcatctgt 1560
   tacgccggcg gtagccggcc agcctcgcag agcaggattc ccgttgagca ccgccaggtg 1620
   cgaataaggg acagtgaaga aggaacaccc gctcgcgggt gggcctactt cacctatcct 1680
   gcccggctga cgccgttgga tacaccaagg aaagtctaca cgaacccttt ggcaaaatcc 1740
   tgtatatcgt gcgaaaaagg atggatatac cgaaaaaatc gctataatga ccccgaagca 1800
   gggttatgca gcggaaaagc gctgcttccc tgctgttttg tggaatatct accgactgga 1860
   aacaggcaaa tgcaggaaat tactgaactg aggggacagg cgagagatct ggcctaggcc 1920
   gaccgaataa atacctgtga cggaagatca cttcgcagaa taaataaatc ctggtgtccc 1980
   tgttgatacc gggaagccct gggccaactt ttggcgaaaa tgagacgttg atcggcacgt 2040
   aagaggttcc aactttcacc ataatgaaat aagatcacta ccgggcgtat tttttgagtt 2100
   gtcgagattt tcaggagcta aggaagctaa aatggagaaa aaaatcactg gatataccac 2160
   cgttgatata tcccaatggc atcgtaaaga acattttgag gcatttcagt cagttgctca 2220
   atgtacctat aaccagaccg ttcagctgga tattacggcc tttttaaaga ccgtaaagaa 2280
   aaataagcac aagttttatc cggcctttat tcacattctt gcccgcctga tgaatgctca 2340
   tccggaatta cgtatggcaa tgaaagacgg tgagctggtg atatgggata gtgttcaccc 2400
   ttgttacacc gttttccatg agcaaactga aacgttttca tcgctctgga gtgaatacca 2460
   cgacgatttc cggcagtttc tacacatata ttcgcaagat gtggcgtgtt acggtgaaaa 2520
   cctggcctat ttccctaaag ggtttattga gaatatgttt ttcgtctcag ccaatccctg 2580
   ggtgagtttc accagttttg atttaaacgt ggccaatatg gacaacttct tcgccccgtt 2640
   ttcaccatgg gcaaatatta tacgcaaggc gacaaggtgc tgatgccgct ggcgattcag 2700
   gttcatcatg ccgtttgtga tggcttccat gtcggcagaa tgcttaatga attacaacag 2760
   tactgcgatg agtggcaggg cggggcgtaa tttttttaag gcagttattg gtgcccataa 2820
   acgcctggtt gctacgcctg aataagtgat aataagcgga tgaatggcag aaattcgaaa 2880
   gcaaattcga cccggtcgtc ggttcagggc agggtcgtta aatagccgct tatgtctatt 2940
   gctggttt 2948
<210> 14
   <211> 24
   <212> DNA
   <213> Vibrio cholerae
<400> 14
   cggtgcaaac gttggaactt tctg 24
<210> 15
   <211> 25
   <212> DNA
   <213> Vibrio cholerae
<400> 15
   ggaaaacaat gccatccaaa ttcgc 25
<210> 16
   <211> 3694
   <212> DNA
   <213> Artificial
<220>
   <223> Synthetic construct
<400> 16
   ttcgatattt tttagttctt taggcccgta gtctgcaaat ccttttatga ttttctatca 60
   aacaaaagag gaaaatagac cagttgcaat ccaaacgaga gtctaataga atgaggtcga 120
   aaagtaaatc gcgcgggttt gttactgata aagcaggcaa gacctaaaat gtgtaaaggg       180
   caaagtgtat actttggcgt caccccttac atattttagg tcttttttta ttgtgcgtaa       240
   ctaacttgcc atcttcaaac aggagggctg gaagaagcag accgctaaca cagtacataa 300
   aaaaggagac atgaacgatg aacatcaaaa agtttgcaaa acaagcaaca gtattaacct 360
   ttactaccgc actgctggca ggaggcgcaa ctcaagcgtt tgcgaaagaa acgaaccaaa 420
   agccatataa ggaaacatac ggcatttccc atattacacg ccatgatatg ctgcaaatcc 480
   ctgaacagca aaaaaatgaa aaatatcaag ttcctgaatt cgattcgtcc acaattaaaa 540
   atatctcttc tgcaaaaggc ctggacgttt gggacagctg gccattacaa aacgctgacg 600
   gcactgtcgc aaactatcac ggctaccaca tcgtctttgc attagccgga gatcctaaaa 660
   atgcggatga cacatcgatt tacatgttct atcaaaaagt cggcgaaact tctattgaca 720
   gctggaaaaa cgctggccgc gtctttaaag acagcgacaa attcgatgca aatgattcta 780
   tcctaaaaga ccaaacacaa gaatggtcag gttcagccac atttacatct gacggaaaaa 840
   tccgtttatt ctacactgat ttctccggta aacattacgg caaacaaaca ctgacaactg 900
   cacaagttaa cgtatcagca tcagacagct ctttgaacat caacggtgta gaggattata 960
   aatcaatctt tgacggtgac ggaaaaacgt atcaaaatgt acagcagttc atcgatgaag 1020
   gcaactacag ctcaggcgac aaccatacgc tgagagatcc tcactacgta gaagataaag 1080
   gccacaaata cttagtattt gaagcaaaca ctggaactga agatggctac caaggcgaag 1140
   aatctttatt taacaaagca tactatggca aaagcacatc attcttccgt caagaaagtc 1200
   aaaaacttct gcaaagcgat aaaaaacgca cggctgagtt agcaaacggc gctctcggta 1260
   tgattgagct aaacgatgat tacacactga aaaaagtgat gaaaccgctg attgcatcta 1320
   acacagtaac agatgaaatt gaacgcgcga acgtctttaa aatgaacggc aaatggtacc 1380
   tgttcactga ctcccgcgga tcaaaaatga cgattgacgg cattacgtct aacgatattt 1440
   acatgcttgg ttatgtttct aattctttaa ctggcccata caagccgctg aacaaaactg 1500
   gccttgtgtt aaaaatggat cttgatccta acgatgtaac ctttacttac tcacacttcg 1560
   ctgtacctca agcgaaagga aacaatgtcg tgattacaag ctatatgaca aacagaggat 1620
   tctacgcaga caaacaatca acgtttgcgc caagcttcct gctgaacatc aaaggcaaga 1680
   aaacatctgt tgtcaaagac agcatccttg aacaaggaca attaacagtt aacaaataaa 1740
   aacgcaaaag aaaatgccga ttgaggccag tttgctcagg ctctccccgt ggaggtaata 1800
   attgacgata tgatcagtaa tacgactcac tagtgggcag atcttcgaat gcatcgcgcg 1860
   caccgtacgt ctcgaggaat tcctgcagga tatctggatc cacgaagctt cccatggtga 1920
   cgtcaccggt tctagatacc taggtgagct ctggtaccct ctagtcaagg cctgtcagcc 1980
   gttaagtgtt cctgtgtcac tgaaaattgc tttgagaggc tctaagggct tctcagtgcg 2040
   ttacatccct ggcttgttgt ccacaaccgt taaaccttaa aagctttaaa agccttatat 2100
   attctttttt ttcttataaa acttaaaacc ttagaggcta tttaagttgc tgatttatat 2160
   taattttatt gttcaaacat gagagcttag tacgtgaaac atgagagctt agtacgttag 2220
   ccatgagagc ttagtacgtt agccatgagg gtttagttcg ttaaacatga gagcttagta 2280
   cgttaaacat gagagcttag tacgtgaaac atgagagctt agtacgtact atcaacaggt 2340
   tgaactgctg atcttcagat ctccgcttgc cctcatctgt tacgccggcg gtagccggcc 2400
   agcctcgcag agcaggattc ccgttgagca ccgccaggtg cgaataaggg acagtgaaga 2460
   aggaacaccc gctcgcgggt gggcctactt cacctatcct gcccggctga cgccgttgga 2520
   tacaccaagg aaagtctaca cgaacccttt ggcaaaatcc tgtatatcgt gcgaaaaagg 2580
   atggatatac cgaaaaaatc gctataatga ccccgaagca gggttatgca gcggaaaagc 2640
   gctgcttccc tgctgttttg tggaatatct accgactgga aacaggcaaa tgcaggaaat 2700
   tactgaactg aggggacagg cgagagatct ggcctaggcc gaccgaataa atacctgtga 2760
   cggaagatca cttcgcagaa taaataaatc ctggtgtccc tgttgatacc gggaagccct 2820
   gggccaactt ttggcgaaaa tgagacgttg atcggcacgt aagaggttcc aactttcacc 2880
   ataatgaaat aagatcacta ccgggcgtat tttttgagtt gtcgagattt tcaggagcta 2940
   aggaagctaa aatggagaaa aaaatcactg gatataccac cgttgatata tcccaatggc 3000
   atcgtaaaga acattttgag gcatttcagt cagttgctca atgtacctat aaccagaccg 3060
   ttcagctgga tattacggcc tttttaaaga ccgtaaagaa aaataagcac aagttttatc 3120
   cggcctttat tcacattctt gcccgcctga tgaatgctca tccggaatta cgtatggcaa 3180
   tgaaagacgg tgagctggtg atatgggata gtgttcaccc ttgttacacc gttttccatg 3240
   agcaaactga aacgttttca tcgctctgga gtgaatacca cgacgatttc cggcagtttc 3300
   tacacatata ttcgcaagat gtggcgtgtt acggtgaaaa cctggcctat ttccctaaag 3360
   ggtttattga gaatatgttt ttcgtctcag ccaatccctg ggtgagtttc accagttttg 3420
   atttaaacgt ggccaatatg gacaacttct tcgcccccgt tttcaccatg ggcaaatatt 3480
   atacgcaagg cgacaaggtg ctgatgccgc tggcgattca ggttcatcat gccgtttgtg 3540
   atggcttcca tgtcggcaga atgcttaatg aattacaaca gtactgcgat gagtggcagg 3600
   gcggggcgta atttttttaa ggcagttatt ggtgcccata aacgcctggt tgctacgcct 3660
   gaataagtga taataagcgg atgaatggca gaaa 3694